# EUROPEAN PATENT APPLICATION

(11) **EP 2 455 080 A1**
(43) Date of publication of application: **23.05.2012**
(21) Application number: 10382312.6
(22) Date of filing: 23.11.2010
(51) Int. Cl.: A61K 31/4245, A61P 25/28

(54) **S1P1 receptor agonists for use in the treatment of multiple sclerosis**

(71) Applicant: Almirall, S.A., 08022 Barcelona (ES)
(72) Inventor: Godessart Marina, Nuria, 08980 Sant Feliu de Llobregat, Barcelona (ES); Tarrason Encuentra, Gema, 08980 Sant Feliu de Llobregat, Barcelona (ES); Aguilar Izquierdo, Nuria, 08980 Sant Feliu de Llobregat, Barcelona (ES)
(74) Representative: Elzaburu Marquez, Alberto

(57) **Abstract**

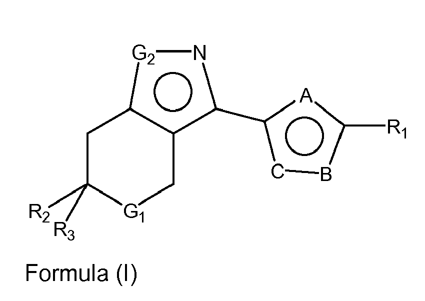

a S1 P1 receptor agonist of formula (I), or a pharmaceutically acceptable salt, or solvate, or N-oxide, or stereoisomer or deuterated derivate thereof, alone or combined with IFNβ, for use in the treatment of multiple sclerosis disease.

## Description

The present invention relates to S1 P1 agonist derivatives of formula (I), alone or combined with interferon beta (IFNβ), for use in the treatment of multiple sclerosis. The present invention also relates to a combination of IFNb and S1P1 agonist of formula (I).

### BACKGROUND OF THE INVENTION

Multiple sclerosis (MS) is a chronic progressive inflammatory disease of the central nervous system in humans. The onset of the disease typically occurs during ages 20 to 40. Women are affected approximately twice as often as men. Multiple sclerosis is an autoimmune disease, in which immune cells of the host attack and destroy the myelin sheath of the neurons in the brain and spinal cord. This causes an impairment in the communication between the brain and other parts of the body, leading to the symptoms of MS. The demyelinated areas appear as plaques and the progression of the disease correlates with the development of new plaques in the portion of the brain or spinal cord controlling the affected areas.

Over time, MS may result in the accumulation of various neurological disabilities. Clinical disability in MS is presumed to be a result of repeated inflammatory injury with subsequent loss of myelin and axons, leading to tissue atrophy.

MS is manifested in physical symptoms (relapses and disability progression), Central Nervous System (CNS) inflammation, brain atrophy and cognitive impairment. Presenting symptoms include focal sensory deficits, focal weakness, visual problems, imbalance and fatigue. Sexual impairment and sphincter dysfunction may occur. Approximately half of the patients with MS may experience cognitive impairment or depression.

Current medications for MS which are disease modifying treatments, i.e. modifying the course of MS, modulate or suppress the immune system.

Betaseron® was the first drug indicated specifically for the treatment of MS. In a major clinical trial, Betaseron® was found to be effective in reducing the number and severity of exacerbations, or relapses, suffered by MS patients, as well as decreasing magnetic resonance imaging (MRI) evidence of MS activity in the brain. Importantly, the results of the trial pertained only to the relapsing- remitting patient group, since other forms of MS were not represented in the trial. Moreover, the trial demonstrated no beneficial effect of the drug on ultimate disability of MS over the 2 to 3 years of the study, and the effectiveness of the drug is significantly impaired by its side effects. One of the side effects of this agent is the elevation of hepatic transaminases (Tremlett HL, Oger J. "Elevated aminotransferases during treatment with interferon-beta for multiple sclerosis: Actions and Outcomes". Mult Scler. 2004 Jun; 10(3):298-301).

Fingolimod (Gilenia®) is the first-in-class sphingosine 1-phosphate (S1P) receptor modulator which has been effective in clinical trials for MS. This compound, a synthetic analog of a natural product derived from the fungus *Isaria sinclairii,* exhibited a peculiar immunomodulatory potential in vivo. When administered to rodents, it caused lymphopenia, due to the sequestration of lymphocytes from the blood into the lymph nodes and Peyer's patches. The close structural similarity of Fingolimod to sphingosine, together with the discovery of the formation of phosphorylated Fingolimod in vivo (FTY720-P) prompted to speculate that FTY720-P could be acting as a mimetic of S1P. This proven to be the case and it was later on demonstrated that FTY720-P binds 4 of the five known S1 P1 receptors, namely S1 P1, S1 P3, S1 P4 and S1 P5.

Expression analysis identified S1 P1 as the dominant S1 P1 receptor expressed on lymphocytes. Moreover, the transfer of S1P1-deficient T cells to normal mice led to the cells being sequestered in lymph nodes, as occurred with animals treated with fingolimod. These two facts strongly pointed out at S1 P1 as the main receptor involved in the lymphopenic effect of Fingolimod in vivo (Baumruker et al, Exp. Opin. Invest. Drugs 2007; 16(3): 283-289). Fingolimod (Gilenia ®) has recently been approved by the FDA on September 2010 as a first oral treatment indicated for the treatment of relapsing-remitting multiple sclerosis. The drug is presumed to act by causing the retention of pathogenic lymphocytes in lymph nodes, thus preventing them to infiltrate the central nervous system (CNS).

The systemic administration of S1P1 agonists prevents the egress of lymphocytes from secondary lymphoid organs, resulting in lymphopenia; that is, a significant reduction in the number of circulating blood lymphocytes. Lymphopenia is believed to be the main factor responsible for the clinical efficacy observed with fingolimod in multiple sclerosis, since it prevents lymphocytes to migrate to the CNS and destroy the myelin sheath. Lymphopenia may lead to an increased susceptibility to opportunistic infections like those caused by herpes virus (Kappos L, et al, "A placebo-controlled trial of oral fingolimod in relapsing multiple sclerosis" N. Engl. J. Med. 2010, 4; 362(5):387-401). In the event of a viral infection during chronic treatment with a S1 P1 agonist, if the infection persists, the treatment has to be stopped to allow the individual to recover lymphocytes in blood to combat the invading pathogen. If recovery is not fast enough, the infected individual may succumb to the infection. This might explain why, in fingolimod clinical trials, two patients died because of herpesvirus infections (Cohen JA, et al. "Oral fingolimod or intramuscular interferon for relapsing multiple sclerosis". N. Engl. J. Med., 2010, 362(5):402-15), since recovery of lymphopenia for this drug has been reported to need some months (Johnson TA. et al., "Reconstitution of circulating lymphocyte counts in FTY720-treated MS patients". Clin. Immunol., 2010, 137(1): 15-20). One of the main factors accounting for the slow recovery of circulating lymphocytes in fingolimod-treated patients is the long half life of the compound in blood (Skerjanec A et al. FTY720, a novel immunomodulator in de novo kidney transplant patients: pharmacokinetics and exposure-response relationship. J.Clin.Pharmacol., 2005, 45(11):1268-78).

In a combination of S1 P1 and interferon beta, a synergy in clinical efficacy compared to any of the treatments alone is expected due to the different mechanism of action of both classes of compounds. Importantly, the antiviral effects reported for interferon beta (Garcia-Montojo M. et al., "Interferon beta treatment: bioavailability and antiviral activity in multiple sclerosis patients", J. Neurovirol., 2007, 3(6):504-12) is expected to help to reduce the incidence of opportunistic viral infections in individuals taking S1 P1 agonists, making this combination more valuable.

### DESCRIPTION OF THE INVENTION

It has been found that the S1 P1 agonists of the present invention do not cause hepatotoxicity in vivo, have a shorter half-life in plasma and a faster recovery of lymphopenia, when compared with Fingolimod. These features render these compounds potentially safer, both as stand-alone therapy for MS patients, or in combination with beta interferons.

Thus, the present invention is directed to a S1 P1 receptor agonist of formula (I) or a pharmaceutically acceptable salt, or solvate, or N-oxide, or stereoisomer or deuterated derivate thereof, for use in the treatment of multiple sclerosis: 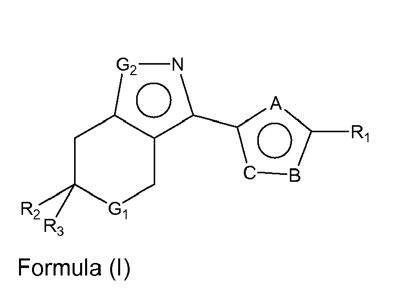 wherein,
either (i) A is selected from the group consisting of -N-, -O- and -S-; B and C are independently selected from the group consisting of -N- and -O-, with the proviso that two of A, B and C are nitrogen atoms, or (ii) two of A, B and C are -N- and one of A, B and C is -NH-;
G¹ is selected from the group consisting of -CH₂-, -NH- and -O-;
G² is selected from the group consisting of -NR⁴- and -O-;
R¹ represents:
➢ a 8 to 10 membered bicyclic N-containing heteroaryl group optionally substituted with a C₁₋₄ carboxyalkyl group or a C₁₋₄ aminoalkyl group,
➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
➢ a pyridone group substituted with one or more C₁₋₄ alkyl groups; C₁₋₄ haloalkyl groups or C₁₋₄ aminoalkyl groups, or
➢ a group of formula: 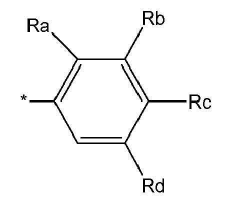 wherein:
   - R^{a} represents a hydrogen atom, a halogen atom, a C₁₋₄ alkyl group, C₃₋₄ cycloalkyl group or a -CF₃ group;
   - R^{b} represents a hydrogen atom, a halogen atom, a C₁₋₄ alkyl group, a -CF₃ group or a C₁₋₄ alkoxy group;
   - R^{d} represents a hydrogen atom, a C₁₋₄ alkyl group or a C₁₋₄ alkoxy group;
   - R^{c} represents:
      o A hydrogen atom, a C₁₋₄ hydroxyalkyl group, a C₁₋₄ aminoalkyl group which is optionally substituted with one or more substituents selected from halogen atoms, hydroxy groups and -CF₃ groups;
      o a 4 to 6-membered saturated N-containing heterocyclic ring optionally substituted with a C₁₋₂ carboxyalkyl group;
      ο -(CH₂)₍₀₋₄₎-C(O)OR', -(CH₂)₍₀₋₄₎-C(O)NR'R", -(CH₂)₍₀₋₄₎-NHC(O)R",
         - S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein,
            ■ R' represents a hydrogen atom or a C₁₋₄ alkyl group,
            ■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
            ■ R' and R" together with the nitrogen atom to which they are attached form a 4 to 6 membered heterocyclic group which contains, as heteroatoms, one N atom and, optionally, one further atom selected from N and O and which is optionally substituted with a carboxy or a C₁₋₄ carboxyalkyl group,,
            or R^{c} together with R^{d} form a C₅₋₆ cycloalkyl group optionally substituted by a
            - NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group. The S1 P1 receptor agonists of formula (I) and methods for their preparation are described in International Patent Application PCT/EP2009/008968, published as WO2010/072352

As used herein the term alkyl embraces optionally substituted, linear or branched hydrocarbon radicals having 1 to 8, preferably 1 to 4 carbon atoms. Examples include methyl, ethyl, n-propyl, i-propyl, n-butyl and tert-butyl radicals.

As used herein, a haloalkyl group is a said alkyl group, for example a C₁₋₄ or C₁₋₂ alkyl group, which is attached to 1, 2 or 3 halogen atoms. The halogen atom is preferably a fluorine atom. Preferably, said haloalkyl group is chosen from -CH₂F -CF₂H, -CF₃ and -CH₂CF₃. -CF₃ and -CH₂CF₃ are preferred.

As used herein, the term hydroxyalkyl embraces linear or branched alkyl radicals having 1 to 4 carbon atoms, any one of which may be substituted with one or more hydroxyl radicals. Examples of such radicals include hydroxymethyl, hydroxyethyl, hydroxypropyl, hydroxybutyl and 1,2-dihydroxypropyl.

As used herein, the term aminoalkyl embraces linear or branched alkyl radicals having 1 to 4 carbon atoms, any one of which may be substituted with one or more amino groups. Examples of such radicals include aminomethyl, aminoethyl, aminopropyl and aminobutyl.

As used herein, the term carboxyalkyl embraces linear or branched alkyl radicals having 1 to 4 carbon atoms, any one of which may be substituted with one or more carboxy radicals. Examples of such radicals include carboxymethyl, carboxyethyl, carboxypropyl, carboxybutyl and 1,2-dicarboxypropyl.

As used herein the term alkoxy embraces optionally substituted, linear or branched oxy-containing radicals each having 1 to 8, preferably, 1 to 4 carbon atoms. Examples include methoxy, ethoxy, *n*-propoxy, *i*-propoxy, *n*-butoxy and *tert*-butoxy radicals.

As used herein, the term aminoalkoxy embraces linear or branched alkoxy radicals having 1 to 4 carbon atoms, any one of which may be substituted with one or more amino groups. Examples of such radicals include aminomethoxy, aminoethoxy, aminopropoxy and aminobutoxy.

As used herein, the term cycloalkyl embraces optionally substituted saturated carbocyclic radicals and, unless otherwise specified, a cycloalkyl radical typically has from 3 to 7, preferably from 3 to 4 carbon atoms. Examples include cyclopropyl, cyclobutyl, cyclopentyl and cyclohexyl. When a cycloalkyl radical carries 2 or more substituents, the substituents may be the same or different. Unless otherwise specified, the substitutents on a cycloalkyl radical are typically themselves unsubstituted.

As used herein, the term heteroaryl radical embraces typically optionally substituted 5-to 10- membered ring systems comprising at least one heteroaromatic ring and containing at least one heteroatom selected from O, S and N. A heteroaryl radical may be a single ring or two or more fused rings wherein at least one ring contains a heteroatom. A said optionally substituted heteroaryl radical is typically unsubstituted or substituted with 1, 2 or 3 substituents which may be the same or different. When a heteroaryl radical carries 2 or more substituents, the substituents may be the same or different. Unless otherwise specified, the substituents on a heteroaryl radical are typically themselves unsubstituted.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1H-pyrazolo[3,4-d]pyrimidinyl, thieno[2,3-d] pyrimidnyl and the various pyrrolopyridyl radicals.

As used herein, the term bicyclic N-containing heteroaryl group is typically an optionally substituted, fused 8 to 10 membered ring system comprising at least one heteroatomic ring, containing a nitrogen atom and optionally one or more, for example, 1, 2 or 3, preferably 1, further heteroatoms selected from O, S and N, preferably N. A said optionally substituted bicyclic N-containing heteroaryl group is typically unsubstituted or substituted with 1, 2 or 3 substituents which may be the same or different. When a heteroaryl radical carries 2 or more substituents, the substituents may be the same or different. Unless otherwise specified, the substituents on a heteroaryl radical are typically themselves unsubstituted.

Example include benzofuranyl, benzoxazolyl, benzimidazolyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, indolinyl, isoindolinyl, isoindolyl, pteridinyl, pyrazolopyrimidinyl, thienopyrimidnyl and pyrrolopyridyl. Pyrrolopyridyl is preferred. 1H-pyrrolo-2,3-b]pyridin-1-yl is more preferred.

As used herein, the term heterocyclic radical embraces typically optionally substituted non-aromatic, saturated or unsaturated C₃-C₁₀ carbocyclic ring systems, preferably C₄-C₆ carbocyclic rings, such as 4, 5 or 6 membered radicals, in which one or more, for example 1, 2, or 3 of the carbon atoms preferably 1 or 2 of the carbon atoms are replaced by a heteroatom selected from N, O and S. Saturated heterocyclic radicals are preferred. A heterocyclic radical may be a single ring or two or more fused rings wherein at least one ring contains a heteroatom. When a heterocyclyl radical carries 2 or more substituents, the substituents may be the same or different. Typically, the substituents on a heterocyclyl radical are themselves unsubstituted, unless otherwise specified.

Examples of heterocyclic radicals include azetidyl, piperidyl, pyrrolidyl, pyrrolinyl, piperazinyl, morpholinyl, thiomorpholinyl, pyrazolinyl, pirazolidinyl, and quinuclidinyl.

As used herein, the term saturated N-containing heterocyclic ring is typically a 4 to 6 membered, optionally substituted heterocyclic radical as defined herein, which is a saturated C₄ to C₆ carbocyclic ring, such as a 4, 5 or 6 membered radical, in which one of the carbon atoms is repaced by N and in which, optionally, one or more, for example 1 or 2, preferably 1 further carbon atom is repaced by a heteroatom selected from N, O and S.

Examples include azetidyl, piperidyl, pyrrolidyl, pyrrolinyl, piperazinyl, morpholinyl, thiomorpholinyl, and pirazolidinyl.

As used herein, some of the atoms, radicals, moieties, chains or cycles present in the general structures of the invention are "optionally substituted". This means that these atoms, radicals, moieties, chains or cycles can be either unsubstituted or substituted in any posiition by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term halogen atom embraces chlorine, fluorine, bromine or iodine atoms typically a fluorine, chlorine or bromine atom, most preferably bromine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge on the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

In one embodiment of the present invention, :
A is selected from the group consisting of -N-, -O- and -S-;
B and C are independently selected from the group consisting of -N- and -O-, with the proviso that at least two of A, B and C are nitrogen atoms;
G¹ is selected from the group consisting of -CH₂-, -NH- and -O-;
G² is selected from the group consisting of -NR⁴- and -O-;
R¹ represents:
   ➢ a 8 to 10 membered bicyclic N-containing heteroaryl group optionally substituted with a C₁₋₄ carboxyalkyl group or a C₁₋₄ aminoalkyl group,
   ➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
   ➢ a pyridone group substituted with one or more C₁₋₄ alkyl groups; C₁₋₄ haloalkyl groups or C₁₋₄ aminoalkyl groups, or
   ➢ a group of formula: 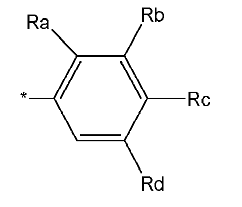
   wherein:
   - R^{a} represents a hydrogen atom, a halogen atom, a C₁₋₄ alkyl group, C₃₋₄ cycloalkyl group or a -CF₃ group;
   - R^{b} represents a hydrogen atom, a halogen atom, a C₁₋₄ alkyl group, a -CF₃ group or a C₁₋₄ alkoxy group;
   - R^{d} represents a hydrogen atom, a C₁₋₄ alkyl group or a C₁₋₄ alkoxy group;
   - R^{c} represents:
      o A hydrogen atom, a C₁₋₄ hydroxyalkyl group, a C₁₋₄ aminoalkyl group which is optionally substituted with one or more substituents selected from halogen atoms, hydroxy groups and -CF₃ groups;
      o a 4 to 6-membered saturated N-containing heterocyclic ring optionally substituted with a C₁₋₂ carboxyalkyl group;
      ο -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or -(CH₂)₍₀₋₃₎-NH-(CH2)₍₁₋₃₎-(NH)₍₀₋₁₎S(O)₂R' wherein,
         ■ R' represents a hydrogen atom or a C₁₋₄ alkyl group,
         ■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
         ■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
         or R^{c} together with R^{d} form a C₅₋₆ cycloalkyl group optionally substituted by a -NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

Typically, in compounds of formula I where R⁴ is a C₃₋₄ cycloalkyl-C₁₋₄ alkyl group, said group is bonded to the nitrogen atom through the alkyl group, i.e. -C₁₋₄ alkyl-C₃₋₄ cycloalkyl.

Typically, when R^{c} represents -(CH₂)₍₀₋₄₎-CONHS(O)₂R', -(CH₂)₍₀₋₄₎-NHS(O)₂R' or -(CH₂)₍₀₋₃₎-NH-(CH₂)₍₁₋₃₎-(NH)₍₀₋₁₎S(O)₂R', then R' is not a hydrogen atom.

Typically, when R^{c} represents -(CH₂)₍₀₋₄₎-NHC(O)R", then R" is not a hydrogen atom.

As used herein, an N-oxide is formed from the tertiary basic amines or imines present in the molecule, using a convenient oxidising agent.

Typically, A is selected from the group consisting of -N- and -O-. Preferably A represents -N-.

More preferably, both A and B represent -N- and C represents -O-.

Typically, G¹ represents a -CH₂- or a -O- group. Preferably G¹ represents a -CH₂-group.

Typically, R² and R³ are independently selected from the group consisting of hydrogen atoms, fluorine atoms and methyl groups. Preferably, both R² and R³ are methyl groups.

Typically, R⁴ is selected from the group consisting of a C₃₋₄ cycloalkyl-C₁₋₄ alkyl group, C₁₋₄ haloalkyl group and a linear or branched unsubstituted C₁₋₄ alkyl group.

Preferably, G² represents -NR⁴-, and R⁴ is selected from the group consisting of a methyl group, ethyl group, t-butyl group, cyclopropylmethyl group and 2,2,2-trifluoroethyl group. More preferably, R⁴ represents a methyl or an ethyl group.

Typically, R¹ represents:
➢ a pyridyl group substituted with one, two or three substituents selected from hydroxy groups and C₁₋₄ alkyl groups;
➢ a pyridone group substituted with one or two C₁₋₂ alkyl groups; or
➢ a group of formula: 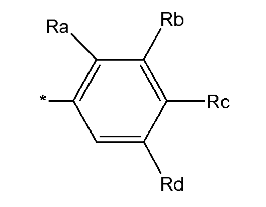 wherein:
   - R^{a} represents a hydrogen atom or a C₁₋₄ alkyl group;
   - R^{b} represents a hydrogen atom or C₁₋₄ alkyl group;
   - R^{d} represents a hydrogen atom or a C₁₋₄ alkyl group;
   - R^{c} represents:
      o a C₁₋₄ hydroxyalkyl group or a C₁₋₄ aminoalkyl substitued by one or more halogen atoms;
      ο -(CH₂)₍₂₋₃₎-C(O)OR', -(CH2)₍₀₋₂₎-C(O)NR'R", -O-(CH₂)₍₂₋₃₎NR'R", -(CH₂)₍₂₋₃₎-NHC(O)R", -S(O)₂NR'R", -(CH₂)₍₀₋₃₎-NR'R" or -(CH₂)₍₁₋₂₎-CONHS(O)₂R' wherein,
         ■ R' represents a hydrogen atom or a methyl group,
         ■ R" represents a hydrogen atom, a C₁₋₄ alkyl group, C₁₋₄ carboxyalkyl group, C₁₋₄ haloalkyl group or a C₁₋₄ hydroxyalkyl group, or
         ■ R' and R" together with the nitrogen atom to which they are attached from a 4 to 6 membered heterocyclic group which contains, as heteroatoms, one N atom and, optionally, one further N atom, and which is optionally substituted with a carboxy or a C₁₋₂ carboxyalkyl group,
or R^{c} together with R^{d} form a cyclohexyl group substituted with a carboxymethylamino group.

Preferably, R¹ represents:
➢ a pyridyl group substituted with two or three substituents selected from hydroxy groups, methyl and ethyl groups, or
➢ a group of formula: 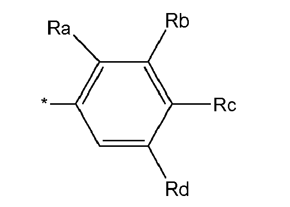 wherein:
   - R^{a} represents a hydrogen atom or a methyl group;
   - R^{b} represents a hydrogen atom, a methyl group;
   - R^{d} represents a hydrogen atom or a methyl group;
   - R^{c} represents: -(CH₂)₍₂₋₃₎-C(O)OR', -(CH₂)₂-C(O)NR'R" or -(CH₂)₍₂₋₃₎-NR'R", wherein
      o R' represents a hydrogen atom;
      o R" represents a hydrogen atom, a C₁₋₂ carboxyalkyl group, a C₁₋₄ haloalkyl group or a C₁₋₂ hydroxyalkyl group; or
      o R' and R" together with the nitrogen atom to which they are attached form a 4 membered saturated heterocyclic group, which contains as heteroatom, one nitrogen atom and which is substituted with a carboxy group.

More preferably, R¹ represents a group of formula: 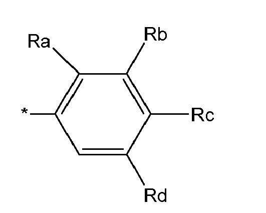 wherein:
o R^{a} represents a hydrogen atom;
ο both R^{b} and R^{d} represent methyl groups; and
o R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group, and C₁₋₂ hydroxyalkyl group.

Typically, G¹ represents a -CH₂- group, G² represents a -NR₄- group, wherein R⁴ represents a methyl or ethyl group, both R² and R³ represent a methyl group, and
R¹ represents:
➢ a pyridyl group substituted with two or three substituents selected from hydroxy groups, methyl or ethyl groups, or
➢ a group of formula: 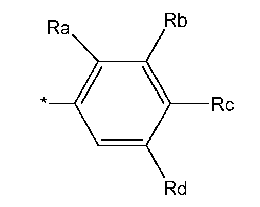 wherein:
   - R^{a} represents a hydrogen atom or a methyl group;
   - R^{b} represents a hydrogen atom, a methyl group,
   - R^{d} represents a hydrogen atom or a methyl group,
   - R^{c} represents: -(CH₂)₍₂₋₃₎-C(O)OR', -(CH₂)₂-C(O)NR'R" or -(CH₂)₍₂₋₃₎-NR'R", wherein:
      o R' represents a hydrogen atom;
      o R" represents a hydrogen atom, a C₁₋₂ carboxyalkyl group, a C₁₋₄ haloalkyl group or a C₁₋₂ hydroxyalkyl group, or
      o R' and R" together with the nitrogen atom to which they are attached form a 4 membered saturated heterocyclic group, which contains as heteroatom, one nitrogen atom and which is substituted with a carboxy group.

More preferably, R¹ represents a group of formula: 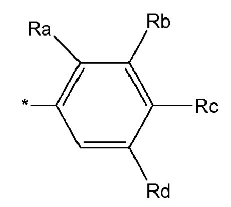 wherein
o R^{a} represents a hydrogen atom;
o both R^{b} and R^{d} represents a methyl group and
o R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group and C₁₋₂ hydroxyalkyl group.

Typically, R¹ represents:
➢ an imidazo[1,2-a]pyridyl group or a 3H-pyrrolo[2,3-b]pyridyl group which are optionally substituted with a carboxyethyl group;
➢ a pyridyl group optionally substituted with one or more substituents selected from hydroxy groups, methyl groups, ethyl groups, carboxyethyl groups, -CF₃ groups, methoxy groups and amino groups
➢ a pyridone group substituted with one or more substituents selected from methyl and ethyl groups; or
➢ a group of formula: 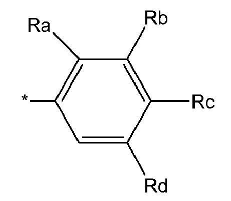 wherein:
   - R^{a} represents a hydrogen atom, a methyl group, cyclopropyl group or a CF₃ group;
   - R^{b} represents a hydrogen atom, a chlorine atom or a methyl group;
   - R^{d} represents a hydrogen atom or a methyl group;
   - R^{c} represents:
      o a C₁₋₄ hydroxyalkyl group or a C₁₋₄ aminoalkyl group substituted with one or more substituents selected from fluorine atoms and hydroxy groups;
      o a 4 to 6-membered saturated N-containing heterocyclic ring which is optionally substituted with a C₁₋₂ carboxyalkyl group
      ο -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R" -(CH₂)₍₀₋₄₎-NR'R", -(CH₂)₍₀₋₄₎-CONHS(O)₂R' -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein
      o R' represents a hydrogen atom or a methyl group,
      o R" represents a hydrogen atom, a methyl group, a cyclopropyl group, a piperidyl group, a C₁₋₂ carboxyalkyl group, a CF₃ group, C₁₋₄ hydroxyalkyl group, or
      o R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a cyclohexyl group optionally substituted by a -NHR^{f} group, wherein R^{f} is selected from the group consisting of a hydrogen atom and a carboxymethyl group;
R² and R³ are independently selected from the group consisting of hydrogen atoms, fluorine atoms and methyl groups; and
R⁴ is selected from the group consisting of a hydrogen atom, a phenyl group, a C₃₋₄ cycloalkyl-C₁₋₂ alkyl group, C₁₋₂ aminoalkyl group, C₁₋₂ haloalkyl group or R⁴ represents a linear or branched C₁₋₄ alkyl group which is optionally substituted by a phenyl group or a pyridyl group.

Typically, R¹ represents a group of formula: 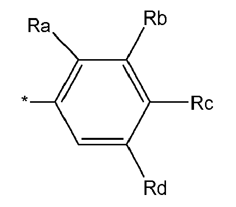 wherein:
- R^{a} represents a hydrogen atom,
- R^{b} represents a methyl group or a CF₃ group,
- R^{d} represents a hydrogen atom or a methyl group;
- R^{c} represents a -(CH₂)₍₀₋₄₎-C(O)NR'R", -(CH₂)₍₀₋₄₎-NHC(O)R" or -(CH₂)₍₀₋₄₎-NR'R", wherein
   o R' represents a hydrogen atom or a methyl group,
   o R" represents a hydrogen atom, a methyl group, a C₁₋₂ carboxyalkyl group or a C₁₋₄ hydroxyalkyl group, or
   o R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group

More preferably, R^{c} represents a -(CH₂)₍₂₋₃₎-NR'R", wherein R' and R" together with the nitrogen atom to which they are attached from a 4 to 6 membered heterocyclic group which contains, as heteroatoms, one N atom, and which is optionally substituted with a carboxy or a C₁₋₂ carboxyalkyl group.

Preferred compounds of the S1 P1 receptor agonist of the invention are represented by the formula (I'), or a pharmaceutically acceptable salt, or solvate, or N-oxide, or stereoisomer or deuterated derivate thereof: 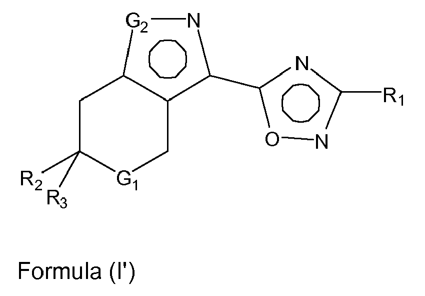 wherein,
G¹ is selected from the group consisting of -CH₂-, and -O-;
G² is selected from the group consisting of -NR⁴- and -O-;
R¹ represents:
➢ A pyrrolopyridyl group, which is unsubstituted or substituted with a C₁₋₂ carboxyalkyl group;
➢ a pyridyl group optionally substituted with 1, 2 or 3 substituents selected from hydroxy groups, C₁₋₂ alkyl groups, C₁₋₂ carboxyalkyl groups, C₁₋₂ haloalkyl groups, C₁₋₂ alkoxy groups, and amino groups;
➢ a pyridone group substituted with 1, 2 or 3 C₁₋₂ alkyl groups; or
➢ a group of formula: 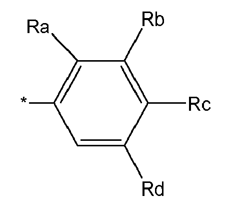 wherein:
   - R^{a} represents a hydrogen atom, a C₁₋₂ alkyl group, a cyclopropyl group or a -CF₃ group;
   - R^{b} represents a hydrogen atom, a chlorine atom, or a C₁₋₂ alkyl group;
   - R^{d} represents a hydrogen atom, or a C₁₋₂ alkyl group;
   - R^{c} represents:
      o a C₁₋₃ hydroxyalkyl group;
      o a carboxyethylpiperazine group;
      ο -(CH₂)₍₀₋₂₎-C(O)OR', -(CH₂)₍₀₋₂₎-C(O)NR'R", -S(O)₂NR'R", or -(CH₂)₍₀₋₄₎-NR'R", wherein,
         ■ R' represents a hydrogen atom,
         ■ R" represents a hydrogen atom, a C₁₋₂ alkyl group, a cyclopropyl group, a C₁₋₂ carboxyalkyl group, a C₁₋₂ haloalkyl group, a C₁₋₂ hydroxyalkyl group or a piperidyl group, or
         ■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₂ carboxyalkyl group,
or R^{c} together with R^{d} forms a cyclohexyl group substituted by a -NHR^{f} group, wherein R^{f} is a carboxymethyl group;
R² and R³ are independently selected from the group consisting of hydrogen atoms, fluorine atoms and C₁₋₂ alkyl groups; and
R⁴ is selected from the group consisting of hydrogen atoms, phenyl groups, cyclopropyl-C₁₋₂ alkyl groups, C₁₋₂ aminoalkyl groups, C₁₋₂ haloalkyl groups and linear or branched C₁₋₄ alkyl groups which are optionally substituted by a phenyl or a pyridyl group,
wherein said pyrrolopyridyl groups are typically 1H-pyrrolo-[2, 3-b]pyridyl groups.

Preferably, the S1 P1 receptor agonist of the present invention is one of the following list:
1. 4-(5-(6,6-Dimethyl-4,5,6,7-tetrahydro-2H-indazol-3-yl)-1,2,4-oxadiazol-3-yl)benzenesulfonamide,
2. (4-(5-(6,6-Dimethyl-4,5,6,7-tetrahydro-2H-indazol-3-yl)-1,2,4-oxadiazol-3-yl)phenyl)methanol,
3. (4-(5-(1,6,6-Trimethyl-4,5,6,7-tetrahydro-1H-indazol-3-yl)-1,2,4-oxadiazol-3-yl)phenyl)methanol,
4. 4-(5-(1,6,6-Trimethyl-4,5,6,7-tetrahydro-1H-indazol-3-yl)-1,2,4-oxadiazol-3-yl)benzenesulfonamide,
5. 4-(5-(1-Ethyl-6,6-dimethyl-4,5,6,7-tetrahydro-1H-indazol-3-yl)-1,2,4-oxadiazol-3-yl)benzenesulfonamide
6. 5-(1-Ethyl-6,6-dimethyl-4,5,6,7-tetrahydro-1H-indazol-3-yl)-3-(pyridin-4-yl)-1,2,4-oxadiazole,
7. 3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid,
8. 3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid,
9. 4-(5-(1-Ethyl-6,6-dimethyl-4,5,6,7-tetrahydro-1H-indazol-3-yl)-1,2,4-oxadiazol-3-yl)benzoic acid,
10. 4-(5-(1-Ethyl-6,6-dimethyl-4,5,6,7-tetrahydro-1H-indazol-3-yl)-1,2,4-oxadiazol-3-yl)benzamide,
11. 5-(1-Ethyl-6,6-dimethyl-4,5,6,7-tetrahydro-1H-indazol-3-yl)-3-(4-(piperazin-1-yl)phenyl)-1,2,4-oxadiazole,
12. 2-(4-(5-(1-Ethyl-6,6-dimethyl-4,5,6,7-tetrahydro-1H-indazol-3-yl)-1,2,4-oxadiazol-3-yl)phenyl)acetic acid,
13. 1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid,
14. 2-(4-(5-(1-Ethyl-6,6-dimethyl-4,5,6,7-tetrahydro-1H-indazol-3-yl)-1,2,4-oxadiazol-3-yl)phenyl)acetamide,
15. 4-(5-(1-Ethyl-6,6-dimethyl-4,5,6,7-tetrahydro-1H-indazol-3-yl)-1,2,4-oxadiazol-3-yl)pyridine 1-oxide,
16. N-(4-(5-(1-Ethyl-6,6-dimethyl-4,5,6,7-tetrahydro-1H-indazol-3-yl)-1,2,4-oxadiazol-3-yl)phenyl)piperidin-4-amine,
17. 5-(1-Ethyl-6,6-dimethyl-4,5,6,7-tetrahydro-1H-indazol-3-yl)-3-(6-methoxypyridin-3-yl)-1,2,4-oxadiazole,
18. 5-(5-(1-Ethyl-6,6-dimethyl-4,5,6,7-tetrahydro-1H-indazol-3-yl)-1,2,4-oxadiazol-3-yl)pyridin-2-ol,
19. 4-(5-(1-Ethyl-6,6-dimethyl-4,5,6,7-tetrahydro-1H-indazol-3-yl)-1,2,4-oxadiazol-3-yl)pyridin-2-ol,
20. 3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide,
21. 4-(5-(1-Benzyl-6,6-dimethyl-4,5,6,7-tetrahydro-1H-indazol-3-yl)-1,2,4-oxadiazol-3-yl)benzamide,
22. 4-(5-(1-tert-Butyl-6,6-dimethyl-4,5,6,7-tetrahydro-1H-indazol-3-yl)-1,2,4-oxadiazol-3-yl)benzamide,
23. 5-(1-Ethyl-6,6-dimethyl-4,5,6,7-tetrahydro-1H-indazol-3-yl)-3-(2-methoxypyridin-4-yl)-1,2,4-oxadiazole,
24. 4-(5-(1-Ethyl-6,6-dimethyl-4,5,6,7-tetrahydro-1H-indazol-3-yl)-1,2,4-oxadiazol-3-yl)-1-methylpyridin-2(1H)-one,
25. 1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid,
26. (4-(5-(1-Ethyl-6,6-dimethyl-4,5,6,7-tetrahydro-1H-indazol-3-yl)-1,2,4-oxadiazol-3-yl)phenyl)methanol,
27. 4-(5-(6,6-Dimethyl-1-phenyl-4,5,6,7-tetrahydro-1H-indazol-3-yl)-1,2,4-oxadiazol-3-yl)benzamide,
28. 3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol,
29. 5-(5-(1-Ethyl-6,6-dimethyl-4,5,6,7-tetrahydro-1H-indazol-3-yl)-1,2,4-oxadiazol-3-yl)-3-methylpyridin-2-ol,
30. 4-(5-(1-Ethyl-6,6-dimethyl-4,5,6,7-tetrahydro-1H-indazol-3-yl)-1,2,4-oxadiazol-3-yl)-3-methylbenzamide,
31. 4-(4-(5-(1-Ethyl-6,6-dimethyl-4,5,6,7-tetrahydro-1H-indazol-3-yl)-1,2,4-oxadiazol-3-yl)benzyl)morpholine,
32. 3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1H)-one,
33. 5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one,
34. N-Cyclopropyl-4-(5-(1-ethyl-6,6-dimethyl-4,5,6,7-tetrahydro-1H-indazol-3-yl)-1,2,4-oxadiazol-3-yl)benzamide,
35. 1-(4-(5-(1-Ethyl-6,6-dimethyl-4,5,6,7-tetrahydro-1H-indazol-3-yl)-1,2,4-oxadiazol-3-yl)phenyl)-N-methylmethanamine,
36. 5-(1-Ethyl-6,6-dimethyl-4,5,6,7-tetrahydro-1H-indazol-3-yl)-3-(3-methylpyridin-4-yl)-1,2,4-oxadiazole,
37. 4-(5-(6,6-Dimethyl-1-(2,2,2-trifluoroethyl)-4,5,6,7-tetrahydro-1H-indazol-3-yl)-1,2,4-oxadiazol-3-yl)benzamide,
38. (4-(5-(1-Ethyl-6,6-dimethyl-4,5,6,7-tetrahydro-1H-indazol-3-yl)-1,2,4-oxadiazol-3-yl)phenyl)methanamine,
39. 5-(1-Ethyl-6,6-dimethyl-4,5,6,7-tetrahydro-1H-indazol-3-yl)-3-(3-methylpyridin-2-yl)-1,2,4-oxadiazole,
40. 2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine,
41. 5-(5-(1-Ethyl-6,6-dimethyl-4,5,6,7-tetrahydro-1H-indazol-3-yl)-1,2,4-oxadiazol-3-yl)-4-methylpyridin-2-ol,
42. 3-(4-(5-(1-Ethyl-6,6-dimethyl-4,5,6,7-tetrahydro-1H-indazol-3-yl)-1,2,4-oxadiazol-3-yl)-3-methylphenyl)propanoic acid,
43. 5-(5-(1-Ethyl-6,6-dimethyl-4,5,6,7-tetrahydro-1H-indazol-3-yl)-1,2,4-oxadiazol-3-yl)-6-methylpyridin-2-ol,
44. 5-(1-Ethyl-6,6-dimethyl-4,5,6,7-tetrahydro-1H-indazol-3-yl)-3-(4-methylpyridin-3-yl)-1,2,4-oxadiazole,
45. 5-(1-Ethyl-6,6-dimethyl-4,5,6,7-tetrahydro-1H-indazol-3-yl)-3-(4-(trifluoromethyl)pyridin-3-yl)-1,2,4-oxadiazole,
46. 5-(1-Ethyl-6,6-dimethyl-4,5,6,7-tetrahydro-1H-indazol-3-yl)-3-(imidazo[1,2-a]pyridin-6-yl)-1,2,4-oxadiazole,
47. 3-(4-(5-(1-Ethyl-6,6-dimethyl-4,5,6,7-tetrahydro-1H-indazol-3-yl)-1,2,4-oxadiazol-3-yl)-3-methylphenyl)propanamide,
48. 2-(4-(5-(1-Ethyl-6,6-dimethyl-4,5,6,7-tetrahydro-1H-indazol-3-yl)-1,2,4-oxadiazol-3-yl)-3-methylphenyl)ethanamine,
49. 3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine,
50. 4-(5-(1-Ethyl-6,6-dimethyl-4,5,6,7-tetrahydro-1H-indazol-3-yl)-1,2,4-oxadiazol-3-yl)-3-(trifluoromethyl)benzoic acid,
51. 4-(5-(1-Ethyl-6,6-dimethyl-4,5,6,7-tetrahydro-1H-indazol-3-yl)-1,2,4-oxadiazol-3-yl)-3-(trifluoromethyl)benzamide,
52. 5-(1-Ethyl-6,6-dimethyl-4,5,6,7-tetrahydro-1H-indazol-3-yl)-3-(2-methylpyridin-3-yl)-1,2,4-oxadiazole,
53. 5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine,
54. 3-Cyclopropyl-4-[5-(1-ethyl-6,6-dimethyl-4,5,6,7-tetrahydro-1H-indazol-3-yl)-1,2,4-oxadiazol-3-yl]benzamide,
55. 5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol,
56. 5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol,
57. 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanoic acid,
58. 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide,
59. 3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol,
60. 3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine,
61. 3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propan-1-amine,
62. 6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine,
63. 2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine,
64. 3-(2,6-Dimethyl-4-(5-(1,6,6-trimethyl-4,5,6,7-tetrahydro-1H-indazol-3-yl)-1,2,4-oxadiazol-3-yl)phenyl)propanoic acid,
65. 3-(2,6-Dimethyl-4-(5-(1,6,6-trimethyl-4,5,6,7-tetrahydro-1H-indazol-3-yl)-1,2,4-oxadiazol-3-yl)phenyl)propanamide,
66. 3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid,
67. 3-(4-(5-(6,6-Dimethyl-4,5,6,7-tetrahydrobenzo[d]isoxazol-3-yl)-1,2,4-oxadiazol-3-yl)-2,6-dimethylphenyl)propanamide,
68. 2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid,
69. 3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine,
70. 3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine,
71. 2-(2,6-Dimethyl-4-(5-(1,6,6-trimethyl-4,5,6,7-tetrahydro-1H-indazol-3-yl)-1,2,4-oxadiazol-3-yl)phenyl)ethanamine,
72. 2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine,
73. 5-(5-(6,6-Dimethyl-1-(pyridin-3-ylmethyl)-4,5,6,7-tetrahydro-1H-indazol-3-yl)-1,2,4-oxadiazol-3-yl)-3-ethyl-6-methylpyridin-2(1H)-one,
74. 2-(Dimethyl-4,5,6,7-tetrahydrobenzo[d]isoxazol-3-yl)-1,2,4-oxadiazol-3-yl)-2,6-dimethylphenethylamino)ethanoic acid,
75. 2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid,
76. 3-(Dimethyl-4,5,6,7-tetrahydrobenzo[d]isoxazol-3-yl)-1,2,4-oxadiazol-3-yl)-2,6-dimethylphenethylamino)propanoic acid,
77. 3-Ethyl-5-(5-(1-ethyl-4,5,6,7-tetrahydro-1H-indazol-3-yl)-1,2,4-oxadiazol-3-yl)-6-methylpyridin-2(1H)-one,
78. 3-Ethyl-6-methyl-5-(5-(1,6,6-trimethyl-4,5,6,7-tetrahydro-1H-indazol-3-yl)-1,2,4-oxadiazol-3-yl)pyridin-2-ol,
79. 5-(5-(6,6-Dimethyl-4,5,6,7-tetrahydrobenzo[d]isoxazol-3-yl)-1,2,4-oxadiazol-3-yl)-3-ethyl-6-methylpyridin-2(1H)-one,
80. 5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol,
81. 3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine,
82. 2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine,
83. 3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid,
84. 3-Ethyl-5-(5-(1-ethyl-6,6-difluoro-4,5,6,7-tetrahydro-1H-indazol-3-yl)-1,2,4-oxadiazol-3-yl)-6-methylpyridin-2(1H)-one,
85. 3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid,
86. 5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one,
87. 3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol,
88. N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine,
89. 2-(2,6-Dimethyl-4-(5-(1,6,6-trimethyl-4,5,6,7-tetrahydro-1H-indazol-3-yl)-1,2,4-oxadiazol-3-yl)phenethylamino)ethanol,
90. 2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid,
91. 1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid,
92. 3-(2-Methyl-4-(5-(1,6,6-trimethyl-4,5,6,7-tetrahydro-1H-indazol-3-yl)-1,2,4-oxadiazol-3-yl)phenyl)propanoic acid,
93. 4-(2,6-Dimethyl-4-(5-(1,6,6-trimethyl-4,5,6,7-tetrahydro-1H-indazol-3-yl)-1,2,4-oxadiazol-3-yl)phenethyl)morpholine,
94. 3-(4-(5-(6,6-Dimethyl-4,5,6,7-tetrahydro-1H-indazol-3-yl)-1,2,4-oxadiazol-3-yl)-2,6-dimethylphenyl)propane-1,2-diol,
95. 3-(4-(5-(1-Ethyl-6,6-dimethyl-4,5,6,7-tetrahydro-1H-indazol-3-yl)-1,2,4-oxadiazol-3-yl)phenylamido)propanoic acid,
96. 3-(4-(5-(6,6-Dimethyl-1-(pyridin-3-ylmethyl)-4,5,6,7-tetrahydro-1H-indazol-3-yl)-1,2,4-oxadiazol-3-yl)-2,6-dimethylphenyl)propane-1,2-diol,
97. 3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid,
98. 3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol,
99. 3-(1-Ethyl-6,6-dimethyl-4,5,6,7-tetrahydro-1H-indazol-3-yl)-5-(pyridin-4-yl)-1,2,4-oxadiazole,
100. 5-(1-Ethyl-6,6-dimethyl-4,5,6,7-tetrahydro-1H-indazol-3-yl)-3-o-tolyl-1,2,4-oxadiazole,
101. 3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid,
102. 3-(5-(5-(6,6-Dimethyl-4,5,6,7-tetrahydrobenzo[d]isoxazol-3-yl)-1,2,4-oxadiazol-3-yl)-1H-pyrrolo[2,3-b]pyridin-1-yl)propanoic acid,
103. 1-amino-3-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
104. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylpropan-2-amine,
105. 3-{4-[3-(1-ethyl-6,6-dimethyl-4,5,6,7-tetrahydro-1H-indazol-3-yl)-1,2,4-oxadiazol-5-yl]-3-methylphenyl}propanoic acid,
106. [2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]amine,
107. 3-(4-{5-[1-(cyclopropylmethyl)-6,6-dimethyl-4,5,6,7-tetrahydro-1H-indazol-3-yl]-1,2,4-oxadiazol-3-yl}-2,6-dimethylphenyl)propanoic acid,
108. (2-{4-[3-(1-ethyl-6,6-dimethyl-4,5,6,7-tetrahydro-1H-indazol-3-yl)-1,2,4-oxadiazol-5-yl]-3-methylphenyl}ethyl)amine,
109. N-[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]glycine,
110. 3-{4-[5-(1-ethyl-6,6-difluoro-4,5,6,7-tetrahydro-1H-indazol-3-yl)-1,2,4-oxadiazol-3-yl]-2,6-dimethylphenyl}propanoic acid,
111. 3-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)propanoic acid,
112. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-hydroxyacetamide,
113. 3-{4-[5-(1-ethyl-6,6-dimethyl-4,5,6,7-tetrahydro-1H-indazol-3-yl)-1,2,4-oxadiazol-3-yl]-2,6-dimethylphenyl}-N-(methylsulfonyl)propanamide,
114. 3-{4-[5-(1-ethyl-6,6-dimethyl-4,5,6,7-tetrahydro-1H-indazol-3-yl)-1,2,4-oxadiazol-3-yl]-2-methylphenyl}propanoic acid,
115. 3-{4-[5-(1-ethyl-6,6-dimethyl-4,5,6,7-tetrahydro-1H-indazol-3-yl)-1,2,4-oxadiazol-3-yl]-2,6-dimethylphenyl}propane-1,2-diol,
116. N-(2-{4-[5-(6,6-dimethyl-4,5,6,7-tetrahydro-1H-indazol-3-yl)-1,2,4-oxadiazol-3-yl]-2,6-dimethylphenyl}ethyl)-2,2,2-trifluoroethanamine,
117. N-({4-[5-(1-ethyl-6,6-dimethyl-4,5,6,7-tetrahydro-1H-indazol-3-yl)-1,2,4-oxadiazol-3-yl]-3-methylphenyl}sulfonyl)-beta-alanine,
118. N-(3-{4-[5-(1-ethyl-6,6-dimethyl-4,5,6,7-tetrahydro-1H-indazol-3-yl)-1,2,4-oxadiazol-3-yl]-2,6-dimethylphenyl}propyl)methanesulfonamide,
119. N-(3-{2,6-dimethyl-4-[5-(1,6,6-trimethyl-4,5,6,7-tetrahydro-1H-indazol-3-yl)-1,2,4-oxadiazol-3-yl]phenyl}propanoyl)glycine,
120. (2-{4-[5-(1-ethyl-6,6-dimethyl-4,5,6,7-tetrahydro-1H-indazol-3-yl)-1,2,4-oxadiazol-3-yl]-2-methylphenyl}ethyl)amine,
121. N-({4-[5-(1-ethyl-6,6-dimethyl-4,5,6,7-tetrahydro-1H-indazol-3-yl)-1,2,4-oxadiazol-3-yl]-3-methylphenyl}sulfonyl)glycine,
122. 1-ethyl-6,6-dimethyl-3-[3-(2-methyl-4-piperidin-4-ylphenyl)-1,2,4-oxadiazol-5-yl]-4,5,6,7-tetrahydro-1H-indazole,
123. (4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)acetic acid,
124. 3-(4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)propanoic acid,
125. (2-{4-[3-(1-ethyl-6,6-dimethyl-4,5,6,7-tetrahydro-1H-indazol-3-yl)-1,2,4-oxadiazol-5-yl]-2,6-dimethylphenoxy}ethyl)amine,
126. 3-{4-[5-(1-ethyl-6,6-dimethyl-4,5,6,7-tetrahydro-1H-indazol-3-yl)-1,2,4-oxadiazol-3-yl]-2,5-dimethylphenyl}propanoic acid,
127. {4-[3-(1-ethyl-6,6-dimethyl-4,5,6,7-tetrahydro-1H-indazol-3-yl)-1,2,4-oxadiazol-5-yl]-2,6-dimethylphenoxylacetic acid,
128. 3-{4-[3-(1-ethyl-6,6-dimethyl-4,5,6,7-tetrahydro-1H-indazol-3-yl)-1,2,4-oxadiazol-5-yl]-2,6-dimethoxyphenyl}propanoic acid,
129. 3-{2-chloro-4-[3-(1-ethyl-6,6-dimethyl-4,5,6,7-tetrahydro-1H-indazol-3-yl)-1,2,4-oxadiazol-5-yl]-6-methoxyphenyl}propanoic acid,
130. (2-{2,6-dimethyl-4-[5-(1,6,6-trimethyl-4,5,6,7-tetrahydro-1H-indazol-3-yl)-1,2,4-oxadiazol-3-yl]phenyl}ethyl)[2-(methylsulfonyl)ethyl]amine,
131. 3-{2-ethyl-4-[5-(1-ethyl-6,6-dimethyl-4,5,6,7-tetrahydro-1H-indazol-3-yl)-1,2,4-oxadiazol-3-yl]-6-methylphenyl}propanoic acid,
132. (2-{3-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
133. {4-[5-(1-ethyl-6,6-dimethyl-4,5,6,7-tetrahydro-1H-indazol-3-yl)-1,2,4-oxadiazol-3-yl]-2,6-dimethylphenyl}acetic acid,
134. [3-({3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}oxy)propyl]amine,
135. 1,6,6-trimethyl-3-[3-(2-methyl-4-piperidin-4-ylphenyl)-1,2,4-oxadiazol-5-yl]-4,5,6,7-tetrahydro-1H-indazole,
136. 2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
137. 2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
138. (2-{3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}ethyl)amine,
139. (2-{2,6-dimethyl-4-[5-(1,6,6-trimethyl-4,5,6,7-tetrahydro-1H-indazol-3-yl)-1,3,4-thiadiazol-2-yl]phenoxy}ethyl)amine,
140. {2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}amine,
141. 2-{2,6-dimethyl-4-[5-(1,6,6-trimethyl-4,5,6,7-tetrahydro-1H-indazol-3-yl)-1,2,4-oxadiazol-3-yl]phenyl}ethanol,
142. 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
143. [1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidin-4-yl]acetic acid,
144. 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
145. (3S)-1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
146. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-alanine,
147. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylalanine,
148. N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-D-alanine,
149. 2-((2,6-dimethyl-4-(5-(1,6,6-trimethyl-4,5,6,7-tetrahydro-1H-indazol-3-yl)-1,2,4-oxadiazol-3-yl)phenethyl)(methyl)amino)acetic acid,
150. (2-{2,6-dimethyl-4-[5-(1,6,6-trimethyl-4,5,6,7-tetrahydro-1H-indazol-3-yl)-1,3,4-oxadiazol-2-yl]phenoxy}ethyl)amine,
151. {2-[4-[5-(1-ethyl-6,6-dimethyl-4,5,6,7-tetrahydro-1H-indazol-3-yl)-1,2,4-oxadiazol-3-yl]-2-(trifluoromethyl)phenoxy]ethyl}amine,
152. (2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
153. (2-{2-(trifluoromethyl)-4-[5-(1,6,6-trimethyl-4,5,6,7-tetrahydro-1H-indazol-3-yl)-1,2,4-oxadiazol-3-yl]phenoxy}ethyl)amine,
154. (2,2-difluoro-2-{2-methyl-4-[5-(1,6,6-trimethyl-4,5,6,7-tetrahydro-1H-indazol-3-yl)-1,2,4-oxadiazol-3-yl]phenyl}ethyl)amine,
155. 1-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
156. 3-{2,6-dimethyl-4-[5-(1,6,6-trimethyl-4,5,6,7-tetrahydro-1H-indazol-3-yl)-1,2,4-oxadiazol-3-yl]phenyl}propan-1-ol,,
157. [4-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperazin-1-yl]acetic acid,
158. 1-(2-{4-[5-(1-ethyl-6,6-dimethyl-4,5,6,7-tetrahydro-1H-indazol-3-yl)-1,2,4-oxadiazol-3-yl]-2,6-dimethylphenyl}ethyl)piperidine-4-carboxylic acid,
159. 1-(2-{4-[5-(1-ethyl-6,6-dimethyl-4,5,6,7-tetrahydro-1H-indazol-3-yl)-1,2,4-oxadiazol-3-yl]-2-methylphenyl}ethyl)piperidine-4-carboxylic acid,
160. 1-(2-{4-[3-(1-ethyl-6,6-dimethyl-4,5,6,7-tetrahydro-1H-indazol-3-yl)-1,2,4-oxadiazol-5-yl]-2-methylphenyl}ethyl)piperidine-4-carboxylic acid,
161. 1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidine-4-carboxylic acid,
162. N-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}glycine,
163. 4-[5-(1,6,6-trimethyl-4,5,6,7-tetrahydro-1H-indazol-3-yl)-1,2,4-oxadiazol-3-yl]benzoic acid,
164. 1-(2-{3-methyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
165. (1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidin-4-yl)acetic acid,
166. 2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethanol,
167. N-{2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethyl}methane-sulfonamide,
168. N-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)-2,2,2-trifluoroethanamine,
169. 1-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)piperidine-4-carboxylic acid,
170. 3-{4-[5-(6,6-dimethyl-4,5,6,7-tetrahydro-1,2-benzisoxazol-3-yl)-1,2,4-oxadiazol-3-yl]-2,6-dimethylphenyl}propane-1,2-diol,
171. 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
172. 2-(2-(trifluoromethyl)-4-(5-(1,6,6-trimethyl-4,5,6,7-tetrahydro-1H-indazol-3-yl)-1,2,4-oxadiazol-3-yl)phenethylamino)acetic acid,
173. 2-(methyl(2-(trifluoromethyl)-4-(5-(1,6,6-trimethyl-4,5,6,7-tetrahydro-1H-indazol-3-yl)-1,2,4-oxadiazol-3-yl)phenethyl)amino)acetic acid,
174. 1-(2-(trifluoromethyl)-4-(5-(1,6,6-trimethyl-4,5,6,7-tetrahydro-1H-indazol-3-yl)-1,2,4-oxadiazol-3-yl)phenethyl)azetidine-3-carboxylic acid,
175. 2-(1-(2-(trifluoromethyl)-4-(5-(1,6,6-trimethyl-4,5,6,7-tetrahydro-1H-indazol-3-yl)-1,2,4-oxadiazol-3-yl)phenethyl)piperidin-4-yl)acetic acid,
176. 1-(2,6-dimethyl-4-(5-(1,6,6-trimethyl-4,5,6,7-tetrahydro-1H-indazol-3-yl)-4H-1,2,4-triazol-3-yl)phenethyl)piperidine-4-carboxylic acid,
177. 1-(2,6-dimethyl-4-(5-(1,6,6-trimethyl-4,5,6,7-tetrahydro-1H-indazol-3-yl)-1,3,4-oxadiazol-2-yl)phenethyl)piperidine-4-carboxylic acid,
178. 1-(2,6-dimethyl-4-(5-(1,6,6-trimethyl-4,5,6,7-tetrahydro-1H-indazol-3-yl)-1,3,4-thiadiazol-2-yl)phenethyl)piperidine-4-carboxylic acid,
179. 2-(1-(2,6-dimethyl-4-(5-(1,6,6-trimethyl-4,5,6,7-tetrahydro-1H-indazol-3-yl)-1,3,4-thiadiazol-2-yl)phenethyl)piperidin-4-yl)acetic acid,
180. 1-(2-(trifluoromethyl)-4-(5-(1,6,6-trimethyl-4,5,6,7-tetrahydro-1H-indazol-3-yl)-1,3,4-oxadiazol-2-yl)phenethyl)piperidine-4-carboxylic acid,
181. 1-(2-(trifluoromethyl)-4-(5-(1,6,6-trimethyl-4,5,6,7-tetrahydro-1H-indazol-3-yl)-1,3,4-thiadiazol-2-yl)phenethyl)piperidine-4-carboxylic acid,
182. 2-(1-(2-(trifluoromethyl)-4-(5-(1,6,6-trimethyl-4,5,6,7-tetrahydro-1H-indazol-3-yl)-1,3,4-thiadiazol-2-yl)phenethyl)piperidin-4-yl)acetic acid,
183. 2-(5-(5-(1-ethyl-6,6-dimethyl-4,5,6,7-tetrahydro-1H-indazol-3-yl)-1,2,4-oxadiazol-3-yl)-1H-pyrrolo[2,3-b]pyridin-1-yl)ethanamine,
184. 5-(1-ethyl-6,6-dimethyl-4,5,6,7-tetrahydro-1H-indazol-3-yl)-3-(1H-pyrrolo[2,3-b]pyridin-5-yl)-1,2,4-oxadiazole,
185. 2-(5-(5-(1,6,6-trimethyl-4,5,6,7-tetrahydro-1H-indazol-3-yl)-1,2,4-oxadiazol-3-yl)-1H-pyrrolo[2,3-b]pyridin-1-yl)ethanamine,
186. 3-(1H-indazol-5-yl)-5-(1,6,6-trimethyl-4,5,6,7-tetrahydro-1H-indazol-3-yl)-1,2,4-oxadiazole,
187. 5-(1-ethyl-6,6-dimethyl-4,5,6,7-tetrahydro-1H-indazol-3-yl)-3-(1H-indol-4-yl)-1,2,4-oxadiazole,
188. 3-(1H-indol-4-yl)-5-(1,6,6-trimethyl-4,5,6,7-tetrahydro-1H-indazol-3-yl)-1,2,4-oxadiazole,
189. 5-(1-ethyl-6,6-dimethyl-4,5,6,7-tetrahydro-1H-indazol-3-yl)-3-(1H-indol-5-yl)-1,2,4-oxadiazole,
190. 3-(1H-benzo[d]imidazol-5-yl)-5-(1-ethyl-6,6-dimethyl-4,5,6,7-tetrahydro-1H-indazol-3-yl)-1,2,4-oxadiazole,
191. 2-(4-(5-(1-ethyl-6,6-dimethyl-4,5,6,7-tetrahydro-1H-indazol-3-yl)-1,2,4-oxadiazol-3-yl)-1H-indol-1-yl)acetic acid,
192. 2-(5-(5-(1,6,6-trimethyl-4,5,6,7-tetrahydro-1H-indazol-3-yl)-1,2,4-oxadiazol-3-yl)-1H-indol-1-yl)acetic acid,
193. 3-(4-(5-(1-ethyl-6,6-dimethyl-4,5,6,7-tetrahydro-1H-indazol-3-yl)-1,2,4-oxadiazol-3-yl)-1H-indol-1-yl)propanoic acid,
194. 2-(5-(5-(1-ethyl-6,6-dimethyl-4,5,6,7-tetrahydro-1H-indazol-3-yl)-1,2,4-oxadiazol-3-yl)-1H-indol-1-yl)acetic acid,
195. 3-(5-(5-(1-ethyl-6,6-dimethyl-4,5,6,7-tetrahydro-1H-indazol-3-yl)-1,2,4-oxadiazol-3-yl)-1H-indol-1-yl)propanoic acid,
196. 2-(1-(2-(trifluoromethyl)-4-(5-(1,6,6-trimethyl-4,5,6,7-tetrahydro-1H-indazol-3-yl)-1,3,4-oxadiazol-2-yl)phenethyl)piperidin-4-yl)acetic acid,
197. 2-(2-(trifluoromethyl)-4-(5-(1,6,6-trimethyl-4,5,6,7-tetrahydro-1H-indazol-3-yl)-1,3,4-thiadiazol-2-yl)phenethylamino)acetic acid,
198. 1-(2-(trifluoromethyl)-4-(5-(1,6,6-trimethyl-4,5,6,7-tetrahydro-1H-indazol-3-yl)-1,3,4-thiadiazol-2-yl)phenethyl)azetidine-3-carboxylic acid,
199. 1-(2-(trifluoromethyl)-4-(3-(1,6,6-trimethyl-4,5,6,7-tetrahydro-1H-indazol-3-yl)-1,2,4-oxadiazol-5-yl)phenethyl)piperidine-4-carboxylic acid
200. 2-(1-(2-(trifluoromethyl)-4-(3-(1,6,6-trimethyl-4,5,6,7-tetrahydro-1H-indazol-3-yl)-1,2,4-oxadiazol-5-yl)phenethyl)piperidin-4-yl)acetic acid
201. 3-(3-(trifluoromethyl)-4-(5-(1,6,6-trimethyl-4,5,6,7-tetrahydro-1H-indazol-3-yl)-1,2,4-oxadiazol-3-yl)phenyl)propanoic acid,
202. 3-(4-(5-(1-ethyl-6,6-dimethyl-4,5,6,7-tetrahydro-1H-indazol-3-yl)-1,2,4-oxadiazol-3-yl)-2-(trifluoromethyl)phenyl)propanoic acid, and
203. 3-(2-(trifluoromethyl)-4-(5-(1,6,6-trimethyl-4,5,6,7-tetrahydro-1H-indazol-3-yl)-1,2,4-oxadiazol-3-yl)phenyl)propanoic acid
or a pharmaceutically acceptable salt, or solvate, or N-oxide, or stereoisomer or deuterated derivate thereof,

More preferably, the S1P1 receptor agonist is one of:
4-(5-(1-Ethyl-6,6-dimethyl-4,5,6,7-tetrahydro-1H-indazol-3-yl)-1,2,4-oxadiazol-3-yl)benzenesulfonamide,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid,
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid,
4-(5-(1-Ethyl-6,6-dimethyl-4,5,6,7-tetrahydro-1H-indazol-3-yl)-1,2,4-oxadiazol-3-yl)benzamide,
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid,
2-(4-(5-(1-Ethyl-6,6-dimethyl-4,5,6,7-tetrahydro-1H-indazol-3-yl)-1,2,4-oxadiazol-3-yl)phenyl)acetamide,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide,
4-(5-(1-Benzyl-6,6-dimethyl-4,5,6,7-tetrahydro-1H-indazol-3-yl)-1,2,4-oxadiazol-3-yl)benzamide,
4-(5-(1-tert-Butyl-6,6-dimethyl-4,5,6,7-tetrahydro-1H-indazol-3-yl)-1,2,4-oxadiazol-3-yl)benzamide,
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol,
5-(5-(1-Ethyl-6,6-dimethyl-4,5,6,7-tetrahydro-1H-indazol-3-yl)-1,2,4-oxadiazol-3-yl)-3-methylpyridin-2-ol,
4-(5-(1-Ethyl-6,6-dimethyl-4,5,6,7-tetrahydro-1H-indazol-3-yl)-1,2,4-oxadiazol-3-yl)-3-methylbenzamide
5-(1-Ethyl-6,6-dimethyl-4,5,6,7-tetrahydro-1H-indazol-3-yl)-3-(3-methylpyridin-4-yl)-1,2,4-oxadiazole,
4-(5-(6,6-Dimethyl-1-(2,2,2-trifluoroethyl)-4,5,6,7-tetrahydro-1H-indazol-3-yl)-1,2,4-oxad iazol-3-yl)benzam ide,
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine,
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid,
5-(5-(1-Ethyl-6,6-dimethyl-4,5,6,7-tetrahydro-1H-indazol-3-yl)-1,2,4-oxadiazol-3-yl)-6-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-methylphenyl)propanamide,
2-(4-(5-(1-Ethyl-6,6-dimethyl-4,5,6,7-tetrahydro-1H-indazol-3-yl)-1,2,4-oxadiazol-3-yl)-3-methylphenyl)ethanamine,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine,
5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol,
6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine,
3-(2,6-Dimethyl-4-(5-(1,6,6-trimethyl-4,5,6,7-tetrahydro-1H-indazol-3-yl)-1,2,4-oxadiazol-3-yl)phenyl)propanamide,
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide,
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid,
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine,
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine,
2-(2,6-Dimethyl-4-(5-(1,6,6-trimethyl-4,5,6,7-tetrahydro-1H-indazol-3-yl)-1,2,4-oxadiazol-3-yl)phenyl)ethanamine,
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine,
2-(Dimethyl-4,5,6,7-tetrahydrobenzo[d]isoxazol-3-yl)-1,2,4-oxadiazol-3-yl)-2,6-dimethylphenethylamino)ethanoic acid,
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid,
3-(Dimethyl-4,5,6,7-tetrahydrobenzo[d]isoxazol-3-yl)-1,2,4-oxadiazol-3-yl)-2,6-dimethylphenethylamino)propanoic acid,
5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one,
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol,
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine,
2-(2,6-Dimethyl-4-(5-(1,6,6-trimethyl-4,5,6,7-tetrahydro-1 H-indazol-3-yl)-1,2,4-oxadiazol-3-yl)phenethylamino)ethanol,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid,
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid,
1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylpropan-2-amine,
3-{4-[3-(1-ethyl-6,6-dimethyl-4,5,6,7-tetrahydro-1H-indazol-3-yl)-1,2,4-oxadiazol-5-yl]-3-methylphenyl}propanoic acid,
[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]amine,
N-({4-[5-(1-ethyl-6,6-dimethyl-4,5,6,7-tetrahydro-1H-indazol-3-yl)-1,2,4-oxadiazol-3-yl]-3-methylphenyl}sulfonyl)-beta-alanine,
2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
(2-{3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}ethyl)amine,
(2-{2,6-dimethyl-4-[5-(1,6,6-trimethyl-4,5,6,7-tetrahydro-1H-indazol-3-yl)-1,3,4-thiadiazol-2-yl]phenoxy}ethyl)amine,
2-{2,6-dimethyl-4-[5-(1,6,6-trimethyl-4,5,6,7-tetrahydro-1H-indazol-3-yl)-1,2,4-oxadiazol-3-yl]phenyl}ethanol,
[1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidin-4-yl]acetic acid
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-alanine,
3-{4-[5-(6,6-dimethyl-4,5,6,7-tetrahydro-1,2-benzisoxazol-3-yl)-1,2,4-oxadiazol-3-yl]-2,6-dimethylphenyl}propane-1,2-diol
5-(1-ethyl-6,6-dimethyl-4,5,6,7-tetrahydro-1H-indazol-3-yl)-3-(1H-indol-4-yl)-1,2,4-oxadiazole,
1-(2-(trifluoromethyl)-4-(5-(1,6,6-trimethyl-4,5,6,7-tetrahydro-1H-indazol-3-yl)-1,3,4-thiadiazol-2-yl)phenethyl)azetidine-3-carboxylic acid and
1-(2-{2-(trifluoromethyl)-4-[5-(1,6,6-trimethyl-4,5,6,7-tetrahydro-1H-indazol-3-yl)-1,2,4-oxadiazol-3-yl)phenyl}ethyl)piperidine-4-carboxylic acid,
   or a pharmaceutically acceptable salt, or solvate, or N-oxide, or stereoisomer or deuterated derivate thereof.

Most preferably, the S1P1 receptor agonist is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated derivate thereof. More preferably the S1P1 receptor agonists are in the form of hydrochloride salt.

In a particularly preferred embodiment, the S1P1 receptor agonist is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

In another perferred embodiment, the S1P1 receptor agonist is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated derivate thereof.

The present invention further provides the use of a S1 P1 receptor agonist of the invention, alone or in combination with IFNβ, for the manufacture of a medicament for the treatment of multiple sclerosis disease. Typically, the S1 P1 receptor agonist is selected from 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or or a pharmaceutically acceptable salt, or solvate, or N-oxide, or stereoisomer or deuterated derivate thereof. More preferably the S1P1 receptor agonists are in the form of hydrochloride salt.

In a particularly preferred embodiment, the S1 P1 receptor agonist used in the manufacture of a medicament for the treatment of multiple sclerosis is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

In another perferred embodiment, the S1 P1 receptor used in the manufacture of a medicament for the treatment of multiple sclerosis is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated derivate thereof.

The present invention also provides a combination of (a) IFNβ and (b) a S1 P1 receptor agonist of formula (I). Typically, the S1 P1 receptor agonist in the combination is selected from 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated derivate thereof. More preferably the S1P1 receptor agonists are in the form of hydrochloride salt.

In a particularly preferred embodiment, the S1 P1 receptor agonist in the combination of the invention is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

In another perferred embodiment, the S1 P1 receptor agonist in the combination of the invention is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, or a pharmaceutically acceptable salt, or solvate, or N-oxide, or stereoisomer or deuterated derivate thereof.

Further provided is a combination as described above which further comprises (c) another compound selected from:
(a) MEK inhibitors, such as ARRY-142886 or ARRY-438162,
(b) immunomodulators such as glatiramer acetate,
(c) inhibitors of DNA synthesis and repair, such as Mitoxantrone,
(d) anti-alpha 4 integrin antibodies, such as Natalizumab (Tysabri),
(e) alpha 4 integrin antagonists such as R-1295 , TBC-4746, CDP-323, ELND-002, Firategrast and TMC-2003,
(f), dyhydrofolate reductase inhibitors, such as Methotrexate or CH-1504,
(g) glucocorticoids such as prednisone or methylprednisolone,
(h) DHODH inhibitors such as Teriflunomide and compounds disclosed in WO 2008/077639 and WO2009021696.
(i) fumaric acid esters, such as *BG-12,*
(j) immunomodulators such as Laquinimod,
(k) anti-CD20 monoclonal antibodies such as Rituximab, Ocrelizumab Ofatumumab or TRU-015,
(l) anti-CD52 such as alemtuzumab,
(m) anti-CD25 such as daclizumab,
(n) anti-CD88, such as eculizumab or pexilizumab,
(o) calcineurin inhibitors such as cyclosporine A or tacrolimus,
(p) IMPDH inhibitors, such as mycophenolate mophetyl,
(q) cannabinoid receptor agonists such as Sativex,
(r) chemokine CCR1 antagonists such as MLN-3897 or PS-031291,
(s) chemokine CCR2 antagonists such as INCB-8696,
(u) NF-kappaB activation inhibitors such as FAE and MLN-0415,
(v) JAK inhibitors such as CP-690550 or INCB018424,
(W) Syk inhibitors, such as R-112,
(x) PKC inhibitors, such as NVP-AEB071,
(y) phosphosdiesterase IV inhibitors such as GRC-4039 and
(z) P38 Inhibitors such as ARRY-797.

Also provided is a product comprising (a) IFNβ and (b) a S1 P1 receptor agonist of the invention, as a combined preparation for simultaneous, separate or sequential use in the treatment of multiple sclerosis. Said product may optionally further comprise an active compound (c), as defined above.

Also provided is a kit of parts comprising (b) a S1 P1 receptor agonist of the invention together with instructions for simultaneous, separate or sequential use in combination with (a) IFNβ, for the treatment of of multiple sclerosis. Said kit may optionally further comprise an active compound (c), as defined above.

Also provided is a package comprising (b) a S1 P1 receptor agonist of the invention and (a) IFNβ, for simultaneous, separate or sequential use in the treatment of multiple sclerosis. Said package may optionally further comprise an active compound (c), as defined above.

Also provided is a use of (b) a S1 P1 receptor agonist of the invention for the preparation of a medicament, for use in combination with (a) IFNβ, for the treatment of multiple sclerosis.

Also provided is a use of (a) IFNβ, for the preparation of a medicament, for use in combination with (b) a S1 P1 receptor agonist of the invention, for the treatment of multiple sclerosis.

Also provided is a use as defined above wherein the IFNβ is for administration at a dosage regime which involves administration of 0.02 to 0.3 mg/week of IFNβ and the S1 P1 receptor agonist is for administration at a dosage regime which involves administration of 0.5 to 2000 mg/day of S1 P1 receptor agonist.

Typically the medicament is for use in treating a human or animal patient suffering or susceptible to hepatic impairment or a condition that would be aggravated by hepatotoxicity. More typically, the said human or animal patient is suffering from liver fibrosis, hepatitis (typically hepatitis A to G), cirrhosis (typically caused by alcoholism) or liver cancer.

The fact that the S1 P1 agonist of the invention have reduced hepatic side effects is a finding of the invention. The present invention therefore also provides the use of a S1 P1 agonist of formula (I), alone or in combination with IFNβ, in the manufacture of a medicament for use in treating or preventing a pathological condition or disease, as defined above, in a human or animal patient which is suffering from or susceptible to hepatic impairment or a condition that would be aggravated by hepatotoxicity.

Also provided is a method of treating a human or animal patient suffering from or susceptible to a pathological condition or disease as defined above, which method comprises simultaneously, separately or sequentially administering to said human or animal patient a therapeutically effective amount of a S1P1 receptor agonist as defined above, alone or in combination with INFβ.

Also provided is a method of treating multiple slcerosis wherein the human or animal patient is suffering from or susceptible to hepatic impairment or a condition that would be aggravated by hepatotoxicity as defined above, which method comprises administering to said human or animal patient a therapeutically effective amount of a S1 P1 receptor agonist as defined abovem, alone or in combination with IFNβ.

The S1 P1 receptor agonist of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compound in the combination, i.e. the sphingosine-1-phosphate agonist of the invention, and the other optional active compounds may be administered together in the same pharmaceutical composition or in different compositions intended for separate, simultaneous, concomitant or sequential administration by the same or a different route.

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2 µg and 150 µg of each therapeutically active ingredient. Alternatively, the active ingredient (s) may be presented without excipients.

Packaging of the formulation for inhalation may be carried out by using suitable inhaler devices such as the Genuair® (formerly known as Novolizer SD2FL) which is described in the following patent applications: WO 97/000703, WO 03/000325 and WO 06/008027.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

The amount of each active which is required to achieve a therapeutic effect will, of course, vary with the particular active, the route of administration, the subject under treatment, and the particular disorder or disease being treated.

In one embodiment of the present invention, the effective doses of the S1 P1 agonist of the present invention is administered vía oral route, while the effective dose of infterferon beta, when it is subministred to the patient as a combined therapy, is administered vía subcutaneous or intramuscular route.

Effective doses are normally in the range of 0.5-2000 mg per day of the active ingredient of the present invention, i.e. the S1 P1 agonist receptor of formula (I). Daily dosage may be administered in one or more treatments, preferably from 1 to 4 treatments, per day. Preferably, the active ingredients are administered once or twice a day. When IFNb is administered as a combined therapy, the effective doses of IFNb are normally in the range of 0.02 to 0.3 mg per week. These doses may be administered once to three times a week, preferably, once or twice a week.

In another embodiment of the invention, the effective dose of both active ingredients, i.e., the S1P1 agonist of formula (I) and IFNβ, may be orally administered as a combined preparation for simultaneous, separate or sequential use in the treatment of multiple sclerosis.

### PHARMACOLOGICAL ACTIVITY

### Example 1: 35S-GTP-g binding assay:

The effect of the compounds was measured using a 35S-GTPyS binding assay. Briefly, membranes were incubated in a buffer containing 20 mM HEPES pH 7.4, 100 mM NaCl, 10 mM MgCl2, 10µM GDP, 50µg/ml saponin and 0.2% fatty acid-free BSA at various concentrations (0.1nM-10µM) and 0.1nM 35S-GTPyS. 10µM S1 P1 was used as 100% maximum efficacy. The assay was incubated for 90 min at room temperature with gentle mixing, and terminated by filtrating the reaction mixture through GF/C filter plates using the Manifold Filtration System. The filters were immediately washed with sodium phosphate pH 7.4 buffers. After drying the filter plates scintillant liquid were added to each well and 35S-GTPyS binding was measured on a Trilux Scintillation Counter.

The results are shown in Table 1.

**Table 1**

| **Compounds** | **EC₅₀ (nM)** |
|---|---|
| 5 | 40 |
| 8 | 17 |
| 21 | 18 |
| 36 | 48 |
| 42 | 16 |
| 48 | 8 |
| 56 | 31 |
| 68 | 1.9 |
| 74 | 1.7 |
| 91 | 16 |
| 97 | 26 |
| 99 | 146 |
| 104 | 8.5 |
| 117 | 11.7 |
| 137 | 3.6 |
| 138 | 13 |
| 139 | 7.3 |
| 141 | 4.6 |
| 142 | 8.7 |
| 143 | 5.1 |
| 146 | 1.4 |
| 162 | 0.56 |
| 170 | 8.3 |
| 171 | 18.8 |
| 187 | 10 |
| 198 | 12 |

### Example 2 - Hepatotoxicity assesment

Acute hepatotoxicity assays were performed in Swiss mice. Animals received a single administration of either vehicle, or Fingolimod or a compound of the present invention (compounds from the list indicated previously) by intraperitoneal route. Twenty-four hours later, animals were sacrificed and the levels of liver markers ALT (alanine aminotransferase), AST (aspartate aminotransferase), and TBIL (total bilirubin) in plasma were determined. An increase in liver markers in this model indicates the potential of that compound to cause liver toxicity in humans at lower doses following longer exposures.

**Table 2: Plasma levels of liver markers of mice 24 h after treatment with either vehicle or test compounds No.142, No.171 or fingolimod.**

| **Compound No.** | **ALT (IU/L)** | **AST (IU/L)** | **TBil (mg/dl)** |
|---|---|---|---|
| Vehicle | 51 ± 9 | 64 ± 9 | 0.5 ± 0.1 |
| 142 (100 mg/kg) | 45 ± 5 | 87 ± 9 | 0.3 ± 0.1 |
| Vehicle | 43 ± 6 | 65 ± 5 | 0.5 ± 0.04 |
| 171 (100 mg/kg) | 42 ± 13 | 66 ± 7 | 0.5 ± 0.04 |
| Vehicle Fingolimod | 62 ± 4 | 100 ± 24 | 0.17 ± 0.04 |
| 30 mg/kg | 59 ± 62 | 354 ± 70* | 0.12 ± 0.01 |
| 100 mg/kg | 355 ± 60** | 589 ± 73*** | 0.16 ± 0.02 |

| | | | |
|---|---|---|---|
| *One-way Anova with Dunet's post test, *p<0.05, **p<0.01, ***p<0.005* | | | |

As it is shown in Table 2, fingolimod-treated mice showed a dose-response increase in AST and ALT levels compared to its corresponding vehicle-treated group, clearly indicating a high hepatotoxicity at the two doses studied. By contrast, the S1 P1 agonist compounds according to the present invention did not cause a significant increase in any of the parameters measured.

The lack of hepatotoxicity observed with Compounds 142 and 171 in this model suggests that the risk of liver toxicity is lower than that of fingolimod. This has implications in monotherapy, when compounds are administered as stand-alone treatments, but the most profound implications are in combination with beta-interferons. Given that beta interferons are also hepatotoxic (Byrnes V et al. "Drug induced liver injury secondary to interferon-beta (IFN-beta) in multiple sclerosis". Ann. Hepatol. 2006, 5:56-59), the coadministration of two substances that cause hepatotoxicity will result in an additive phenomenon, inducing a higher liver toxicity than any of the two agents alone. Thus, the highest advantage of compounds of the present invention versus fingolimod is in their combination with interferon beta, where they are expected to have a lower potential of hepatotoxicity than the combination of interferons with fingolimod.

### Example 3: Half life in rats after intravenous administration.

Rats were administered with 1 mg/kg of test compounds by intraveneous route to Wistar rats. Blood samples were collected from retroorbital plexus at several time points after administration and plasma levels of compounds were quantified by MS-HPLC.

In samples of rats treated with fingolimod, both the parental compound (fingolimod) and the active metabolite (fingolimod-phosphate) were determined.

**Table3: Pharmacokinetic parameters of fingolimod, Compounds No. 142 and No. 171 in rats following i.v. administration of 1 mg/kg.**

| Compound No. | **Cmax(ng/ml) Plasma** | **Tmax (h)** | **AUC 0-24 (ng.h/ml)** | **Half life t 1/2 (h)** |
|---|---|---|---|---|
| Fingolimod* | 50.5 | 0.1 | 279 | 26.1 |
| Fingolimod-P | 92.1 | 1 | 1329 | 24.4 |
| 142 | 824 | 0.1 | 2627 | 3.5 |
| 171 | 986 | 0.1 | 6227 | 8.2 |

| | | | | |
|---|---|---|---|---|
| *Animals were administered with the parental compound, fingolimod, and values from both fingolimod and its in vivo generated active metabolite, fingolimod-phosphate, have been measured. | | | | |

The advantage of compound No. 142 or compound No. 171 is that they have a shorter half life than fingolimod and this is translated into a faster recovery of lymphopenia (Figure 1). In the event of an unexpected toxicity leading to treatment discontinnuation, the clearance of the compound and thus the dissappearance of the side effect will be faster with compounds of the present invention than that with Fingolimod.

### Example 3. Recovery of lymphopenia in rats after a single oral administration

Test compounds were administered by oral route to Wistar rats at a dose of each compound that caused maximal lymphopenia. At several times after administration, animals were anestesized and blood extracted from retroorbital plexus and collected into heparinized tubes. Total lymphocyte numbers were determined using a Sysmex K-800 haemocytometer.

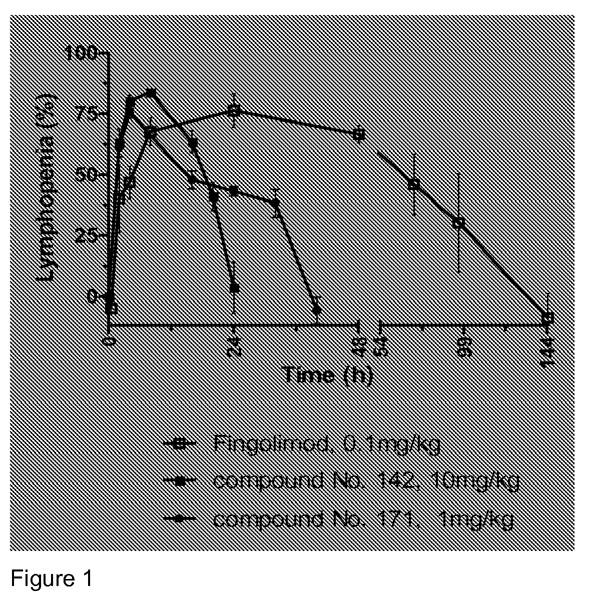

In agreement with their shorter half lives (Table 3), animals treated with compounds Nos. 142 and 171 recover lymphocytes in blood between 24 and 40h after administration. However, 144h are needed for the same effect to occur with fingolimod. Thus, patients treated with compounds of the present invention will recover their immunocompetent status in a more shorter time than in the case of fingolimod when the treatment has to be discontinued due to an opportunistic infection.

## Claims

1. A S1 P1 receptor agonist of formula (I), or a pharmaceutically acceptable salt, or solvate, or N-oxide, or stereoisomer or deuterated derivate thereof, for use in the treatment of multiple sclerosis: 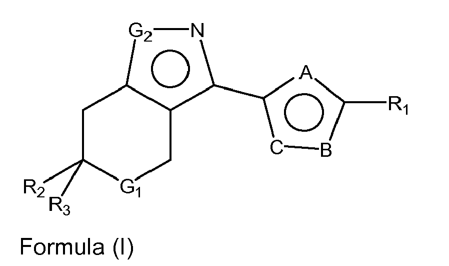 wherein
either (i) A is selected from the group consisting of -N-, -O- and -S-; B and C are independently selected from the group consisting of -N- and -O-, with the proviso that two of A, B and C are nitrogen atoms, or (ii) two of A, B and C are -N- and one of A, B and C is -NH-;
G¹ is selected from the group consisting of -CH₂-, -NH- and -O-;
G² is selected from the group consisting of -NR⁴- and -O-;
R¹ represents:
➢ a 8 to 10 membered bicyclic N-containing heteroaryl group optionally substituted with a C₁₋₄ carboxyalkyl group or a C₁₋₄ aminoalkyl group,
➢ a pyridyl group optionally substituted with one or more substituents selected from hydroxy groups, C₁₋₄ alkyl groups, C₁₋₄ carboxyalkyl groups, C₁₋₄ haloalkyl groups, C₁₋₄ alkoxy groups, amino groups, C₁₋₄ aminoalkyl groups and C₁₋₄ aminoalkoxy groups,
➢ a pyridone group substituted with one or more C₁₋₄ alkyl groups; C₁₋₄ haloalkyl groups or C₁₋₄ aminoalkyl groups, or
➢ a group of formula: 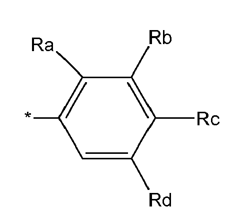 wherein:
• R^{a} represents a hydrogen atom, a halogen atom, a C₁₋₄ alkyl group, C₃₋₄ cycloalkyl group or a -CF₃ group;
• R^{b} represents a hydrogen atom, a halogen atom, a C₁₋₄ alkyl group, a -CF₃ group or a C₁₋₄ alkoxy group;
• R^{d} represents a hydrogen atom, a C₁₋₄ alkyl group or a C₁₋₄ alkoxy group;
• R^{c} represents:
o a hydrogen atom, a C₁₋₄ hydroxyalkyl group, a C₁₋₄ aminoalkyl group which is optionally substituted with one or more substituents selected from halogen atoms, hydroxy groups and -CF₃ groups;
o a 4 to 6-membered saturated N-containing heterocyclic ring optionally substituted with a C₁₋₂ carboxyalkyl group;
o -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R", -(CH₂)₍₀₋₄₎-NR'R", -(CH₂)₍₀₋₄₎-CONHS(O)₂R', -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein,
■ R' represents a hydrogen atom or a C₁₋₄ alkyl group,
■ R" represents a hydrogen atom, a C₁₋₄ alkyl group, a C₃₋₄ cycloalkyl group, a C₁₋₄ carboxyalkyl group, a C₁₋₄ haloalkyl group, a C₁₋₄ hydroxyalkyl group or a 6 membered, saturated N-containing heterocyclic ring, or
■ R' and R" together with the nitrogen atom to which they are attached form a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a C₅₋₆ cycloalkyl group optionally substituted by a -NHR^{f} group, wherein R^{f} is selected from the group consisting of a hydrogen atom and a carboxymethyl group;
R² and R³ are independently selected from the group consisting of hydrogen atoms, halogen atoms and C₁₋₄ alkyl groups; and
R⁴ is selected from the group consisting of a hydrogen atom, a phenyl group, a C₃₋₄ cycloalkyl-C₁₋₄ alkyl group, C₁₋₄ aminoalkyl group, C₁₋₄ haloalkyl group and a linear or branched C₁₋₄ alkyl group which is optionally substituted by a phenyl or a pyridyl group.

2. The S1P1 receptor agonist according to claim 1, wherein A is selected from the group consisting of -N- and -O-.

3. The S1 P1 receptor agonist according to claim 2, wherein A represents -N-.

4. The S1 P1 receptor agonist according to any one of claims 1 to 3, wherein both A and B represent -N- and C represents -O-.

5. The S1 P1 receptor agonist according to any one of the preceding claims, wherein G¹ represents a -CH₂- or a -O- group.

6. The S1 P1 receptor agonist according to claim 5, wherein G¹ represents a -CH₂-group.

7. The S1 P1 receptor agonist according to any one of the preceding claims, wherein R² and R³ are independently selected from the group consisting of hydrogen atoms, fluorine atoms and methyl groups.

8. The S1 P1 receptor agonist according to claim 7, wherein both R² and R³ are methyl groups.

9. The S1 P1 receptor agonist according to any one of claims 1 to 8, wherein R⁴ is selected from the group consisting of a C₃₋₄ cycloalkyl-C₁₋₄ alkyl group, C₁₋₄ haloalkyl group and a linear or branched unsubstituted C₁₋₄ alkyl group.

10. The S1 P1 receptor agonist according to claim 9, wherein G² represents-NR⁴-, and wherein R⁴ is selected from the group consisting of a methyl group, ethyl group, t-butyl group, cyclopropylmethyl group and 2,2,2-trifluoroethyl group.

11. The S1 P1 receptor agonist according to claim 10, wherein R⁴ represents a methyl or an ethyl group.

12. The S1P1 receptor agonist according to any one of the preceding claims, wherein R¹ represents:
➢ a pyridyl group substituted with one, two or three substituents selected from hydroxy groups and C₁₋₄ alkyl groups;
➢ a pyridone group substituted with one or two C₁₋₂ alkyl groups; or
➢ a group of formula: 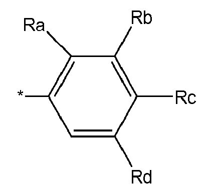 wherein:
• R^{a} represents a hydrogen atom or a C₁₋₄ alkyl group;
• R^{b} represents a hydrogen atom or C₁₋₄ alkyl group;
• R^{d} represents a hydrogen atom or a C₁₋₄ alkyl group;
• R^{c} represents:
○ a C₁₋₄ hydroxyalkyl group or a C₁₋₄ aminoalkyl substitued by one or more halogen atoms;
○ -(CH₂)₍₂₋₃₎-C(O)OR', -(CH₂)₍₀₋₂₎-C(O)NR'R", -O-(CH₂)₍₂₋₃₎NR'R", -(CH₂)₍₂₋₃₎-NHC(O)R", -S(O)₂NR'R", -(CH₂)₍₀₋₃₎-NR'R" or -(CH₂)₍₁₋₂₎-CONHS(O)₂R' wherein,
■ R' represents a hydrogen atom or a methyl group,
■ R" represents a hydrogen atom, a C₁₋₄ alkyl group, C₁₋₄ carboxyalkyl group, C₁₋₄ haloalkyl group or a C₁₋₄ hydroxyalkyl group, or
■ R' and R" together with the nitrogen atom to which they are attached from a 4 to 6 membered heterocyclic group which contains, as heteroatoms, one N atom and, optionally, one further N atom, and which is optionally substituted with a carboxy or a C₁₋₂ carboxyalkyl group,
or R^{c} together with R^{d} form a cyclohexyl group substituted with a carboxymethylamino group.

13. The S1 P1 receptor agonist according to claim 12, wherein R¹ represents:
➢ a pyridyl group substituted with two or three substituents selected from hydroxy groups, methyl and ethyl groups, or
➢ a group of formula: 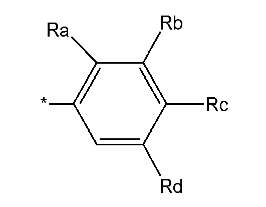 wherein:
• R^{a} represents a hydrogen atom or a methyl group;
• R^{b} represents a hydrogen atom, a methyl group;
• R^{d} represents a hydrogen atom or a methyl group;
• R^{c} represents: -(CH₂)₍₂₋₃₎-C(O)OR', -(CH₂)₂-C(O)NR'R" or -(CH₂)₍₂₋₃₎-NR'R", wherein
o R' represents a hydrogen atom;
o R" represents a hydrogen atom, a C₁₋₂ carboxyalkyl group, a C₁₋₄ haloalkyl group or a C₁₋₂ hydroxyalkyl group; or
o R' and R" together with the nitrogen atom to which they are attached form a 4 membered saturated heterocyclic group, which contains as heteroatom, one nitrogen atom and which is substituted with a carboxy group.

14. The S1P1 receptor agonist according to claim 13, wherein R¹ represents a group of
formula: 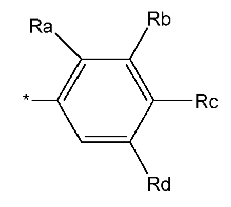 wherein:
○ R^{a} represents a hydrogen atom;
○ both R^{b} and R^{d} represent methyl groups; and
○ R^{c} represents -(CH₂)₍₂₋₃₎-C(O)OH or -(CH₂)₍₂₋₃₎-NHR", wherein R" is selected from a hydrogen atom, a C₁₋₂ carboxyalkyl group, and C₁₋₂ hydroxyalkyl group.

15. The S1 P1 receptor agonist according to any one of the preceding claims wherein:
G¹ represents a -CH₂- group,
G² represents a -NR₄- group, wherein R⁴ represents a methyl or ethyl group,
both R² and R³ represent a methyl group, and
R¹ represents:
➢ a pyridyl group substituted with two or three substituents selected from hydroxy groups, methyl or ethyl groups, or
➢ a group of formula: 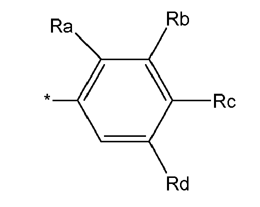 wherein:
• R^{a} represents a hydrogen atom or a methyl group;
• R^{b} represents a hydrogen atom, a methyl group,
• R^{d} represents a hydrogen atom or a methyl group,
• R^{c} represents: -(CH₂)₍₂₋₃₎-C(OOR', -(CH₂)₂-C(O)NR'R" or -(CH₂)₍₂₋₃₎-NR'R", wherein:
○ R' represents a hydrogen atom;
○ R" represents a hydrogen atom, a C₁₋₂ carboxyalkyl group, a C₁₋₄ haloalkyl group or a C₁₋₂ hydroxyalkyl group, or
○ R' and R" together with the nitrogen atom to which they are attached form a 4 membered saturated heterocyclic group, which contains as heteroatom, one nitrogen atom and which is substituted with a carboxy group.

16. The S1P1 receptor agonist according to claim 15, wherein R¹ represents a group of formula: 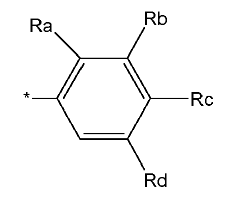 wherein
o R^{a} represents a hydrogen atom;
o both R^{b} and R^{d} represents a methyl group and
o R^{c} represents -(CH₂)₍₂₋₃₎-C(OOH or -(CH₂)₍₂₋₃₎-NHR", wherein R" is selected from a hydrogen atom, a C₁₋₂ carboxyalkyl group and C₁₋₂ hydroxyalkyl group.

17. The S1 P1 receptor agonist of according to claim 1, wherein R¹ represents:
➢ an imidazo[1,2-a]pyridyl group or a 3H-pyrrolo[2,3-b]pyridyl group which are optionally substituted with a carboxyethyl group;
➢ a pyridyl group optionally substituted with one or more substituents selected from hydroxy groups, methyl groups, ethyl groups, carboxyethyl groups, -CF₃ groups, methoxy groups and amino groups
➢ a pyridone group substituted with one or more substituents selected from methyl and ethyl groups; or
➢ a group of formula: 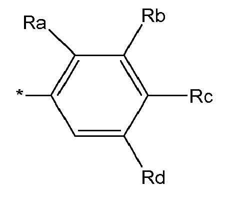 wherein:
• R^{a} represents a hydrogen atom, a methyl group, cyclopropyl group or a CF₃ group;
• R^{b} represents a hydrogen atom, a chlorine atom or a methyl group;
• R^{d} represents a hydrogen atom or a methyl group;
• R^{c} represents:
○ a C₁₋₄ hydroxyalkyl group or a C₁₋₄ aminoalkyl group substituted with one or more substituents selected from fluorine atoms and hydroxy groups;
○ a 4 to 6-membered saturated N-containing heterocyclic ring which is optionally substituted with a C₁₋₂ carboxyalkyl group
○ -(CH₂)₍₀₋₄₎-C(O)OR', -(CH₂)₍₀₋₄₎-C(O)NR'R", -(CH₂)₍₀₋₄₎-NHC(O)R", -S(O)₂NR'R", -O-(CH₂)₍₂₋₄₎NR'R", -O-(CH₂)₍₁₋₄₎C(O)OR", -O-(CH₂)₍₁₋₄₎-C(O)NR'R" -(CH₂)₍₀₋₄₎-NR'R", -(CH₂)₍₀₋₄₎-CON HS(O)₂R' -(CH₂)₍₀₋₄₎-NHS(O)₂R' or -(CH₂)₍₀₋₃₎-NH-(CH₂)₍₁₋₃₎-(NH)₍₀₋₁₎S(O)₂R' wherein
○ R' represents a hydrogen atom or a methyl group,
○ R" represents a hydrogen atom, a methyl group, a cyclopropyl group, a piperidyl group, a C₁₋₂ carboxyalkyl group, a CF₃ group, C₁₋₄ hydroxyalkyl group, or
○ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group,
or R^{c} together with R^{d} form a cyclohexyl group optionally substituted by a -NHR^{f} group, wherein R^{f} is selected from the group consisting of a hydrogen atom and a carboxymethyl group;
R² and R³ are independently selected from the group consisting of hydrogen atoms, fluorine atoms and methyl groups; and
R⁴ is selected from the group consisting of a hydrogen atom, a phenyl group, a C₃₋₄ cycloalkyl-C₁₋₂ alkyl group, C₁₋₂ aminoalkyl group, C₁₋₂ haloalkyl group or R⁴ represents a linear or branched C₁₋₄ alkyl group which is optionally substituted by a phenyl group or a pyridyl group.

18. The S1P1 receptor agonist according to any one of claims 1 to 17, wherein R¹ represents a group of formula: 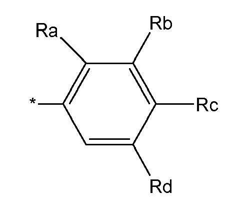 wherein:
• R^{a} represents a hydrogen atom,
• R^{b} represents a methyl group or a CF₃ group,
• R^{d} represents a hydrogen atom or a methyl group;
• R^{c} represents a -(CH₂)₍₀₋₄₎-C(O)NR'R", -(CH₂)₍₀₋₄₎-NHC(O)R" or -(CH₂)₍₀₋₄₎-NR'R", wherein
○ R' represents a hydrogen atom or a methyl group,
○ R" represents a hydrogen atom, a methyl group, a C₁₋₂ carboxyalkyl group or a C₁₋₄ hydroxyalkyl group, or
○ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₄ carboxyalkyl group

19. The S1 P1 receptor agonist according to claim 18, wherein R^{c} represents a -(CH₂)₍₂₋₃)-NR'R", wherein R' and R" together with the nitrogen atom to which they are attached from a 4 to 6 membered heterocyclic group which contains, as heteroatoms, one N atom, and which is substituted with a carboxy or a C₁₋₂ carboxyalkyl group.

20. The S1P1 receptor agonist according to claim 1, which is a compound of general formula (I'), or a pharmaceutically acceptable salt or N-oxide thereof: 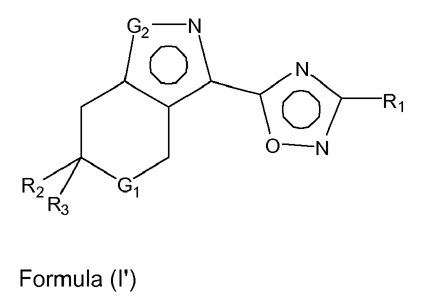 wherein,
G¹ is selected from the group consisting of -CH₂-, and -O-;
G² is selected from the group consisting of -NR⁴- and -O-;
R¹ represents:
➢ a pyrrolopyridyl group, which is unsubstituted or substituted with a C₁₋₂ carboxyalkyl group;
➢ a pyridyl group optionally substituted with 1, 2 or 3 substituents selected from hydroxy groups, C₁₋₂ alkyl groups, C₁₋₂ carboxyalkyl groups, C₁₋₂ haloalkyl groups, C₁₋₂ alkoxy groups, and amino groups;
➢ a pyridone group substituted with 1, 2 or 3 C₁₋₂ alkyl groups; or
➢ a group of formula: 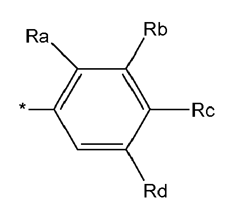 wherein:
• R^{a} represents a hydrogen atom, a C₁₋₂ alkyl group, a cyclopropyl group or a -CF₃ group;
• R^{b} represents a hydrogen atom, a chlorine atom, or a C₁₋₂ alkyl group;
• R^{d} represents a hydrogen atom, or a C₁₋₂ alkyl group;
• R^{c} represents:
○ a C₁₋₃ hydroxyalkyl group;
○ a carboxyethylpiperazine group;
○ -(CH₂)₍₀₋₂₎-C(O)OR', -(CH₂)₍₀-₂₎-C(O)NR'R", -S(O)₂NR'R", or -(CH₂)₍₀₋₄₎-NR'R", wherein,
■ R' represents a hydrogen atom,
■ R" represents a hydrogen atom, a C₁₋₂ alkyl group, a cyclopropyl group, a C₁₋₂ carboxyalkyl group, a C₁₋₂ haloalkyl group, a C₁₋₂ hydroxyalkyl group or a piperidyl group, or
■ R' and R" together with the nitrogen atom to which they are attached from a 4 to 6 membered heterocyclic group which contains, as heteroatoms, one N atom and, optionally, one further atom selected from N and O, and which is optionally substituted with a carboxy or a C₁₋₂ carboxyalkyl group,
or R^{c} together with R^{d} forms a cyclohexyl group substituted by a -NHR^{f} group, wherein R^{f} is a carboxymethyl group;
R² and R³ are independently selected from the group consisting of hydrogen atoms, fluorine atoms and C₁₋₂ alkyl groups; and
R⁴ is selected from the group consisting of hydrogen atoms, phenyl groups, cyclopropyl-C₁₋₂ alkyl groups, C₁₋₂ aminoalkyl groups, C₁₋₂ haloalkyl groups and linear or branched C₁₋₄ alkyl groups which are optionally substituted by a phenyl or a pyridyl group.

21. The S1 P1 receptor agonist according to claim 1, which is one of:
4-(5-(6,6-Dimethyl-4,5,6,7-tetrahydro-2H-indazol-3-yl)-1,2,4-oxadiazol-3-yl)benzenesulfonamide,
(4-(5-(6,6-Dimethyl-4,5,6,7-tetrahydro-2H-indazol-3-yl)-1,2,4-oxadiazol-3-yl)phenyl)methanol,
(4-(5-(1,6,6-Trimethyl-4,5,6,7-tetrahydro-1H-indazol-3-yl)-1,2,4-oxadiazol-3-yl)phenyl)methanol,
4-(5-(1,6,6-Trimethyl-4,5,6,7-tetrahydro-1H-indazol-3-yl)-1,2,4-oxadiazol-3-yl)benzenesulfonamide,
4-(5-(1-Ethyl-6,6-dimethyl-4,5,6,7-tetrahydro-1H-indazol-3-yl)-1,2,4-oxadiazol-3-yl)benzenesulfonamide,
5-(1-Ethyl-6,6-dimethyl-4,5,6,7-tetrahydro-1H-indazol-3-yl)-3-(pyridin-4-yl)-1,2,4-oxadiazole,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanoic acid,
3-(4-(4-(5-(1-Ethyl-6,6-dimethyl-4,5,6,7-tetrahydro-1H-indazol-3-yl)-1,2,4-oxadiazol-3-yl)phenyl)piperazin-1-yl)propanoic acid,
4-(5-(1-Ethyl-6,6-dimethyl-4,5,6,7-tetrahydro-1H-indazol-3-yl)-1,2,4-oxadiazol-3-yl)benzoic acid,
4-(5-(1-Ethyl-6,6-dimethyl-4,5,6,7-tetrahydro-1H-indazol-3-yl)-1,2,4-oxadiazol-3-yl)benzamide,
5-(1-Ethyl-6,6-dimethyl-4,5,6,7-tetrahydro-1H-indazol-3-yl)-3-(4-(piperazin-1-yl)phenyl)-1,2,4-oxadiazole,
2-(4-(5-(1-Ethyl-6,6-dimethyl-4,5,6,7-tetrahydro-1H-indazol-3-yl)-1,2,4-oxadiazol-3-yl)phenyl)acetic acid,
1-(4-(5-(1-Ethyl-6,6-dimethyl-4,5,6,7-tetrahydro-1H-indazol-3-yl)-1,2,4-oxadiazol-3-yl)benzyl)azetidine-3-carboxylic acid,
2-(4-(5-(1-Ethyl-6,6-dimethyl-4,5,6,7-tetrahydro-1H-indazol-3-yl)-1,2,4-oxadiazol-3-yl)phenyl)acetamide,
4-(5-(1-Ethyl-6,6-dimethyl-4,5,6,7-tetrahydro-1H-indazol-3-yl)-1,2,4-oxadiazol-3-yl)pyridine 1-oxide,
N-(4-(5-(1-Ethyl-6,6-dimethyl-4,5,6,7-tetrahydro-1H-indazol-3-yl)-1,2,4-oxadiazol-3-yl)phenyl)piperidin-4-amine,
5-(1-Ethyl-6,6-dimethyl-4,5,6,7-tetrahydro-1H-indazol-3-yl)-3-(6-methoxypyridin-3-yl)-1,2,4-oxadiazole,
5-(5-(1-Ethyl-6,6-dimethyl-4,5,6,7-tetrahydro-1H-indazol-3-yl)-1,2,4-oxadiazol-3-yl)pyridin-2-ol,
4-(5-(1-Ethyl-6,6-dimethyl-4,5,6,7-tetrahydro-1H-indazol-3-yl)-1,2,4-oxadiazol-3-yl)pyridin-2-ol,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propanamide,
4-(5-(1-Benzyl-6,6-dimethyl-4,5,6,7-tetrahydro-1H-indazol-3-yl)-1,2,4-oxadiazol-3-yl)benzamide,
4-(5-(1-tert-Butyl-6,6-dimethyl-4,5,6,7-tetrahydro-1H-indazol-3-yl)-1,2,4-oxadiazol-3-yl)benzamide,
5-(1-Ethyl-6,6-dimethyl-4,5,6,7-tetrahydro-1H-indazol-3-yl)-3-(2-methoxypyridin-4-yl)-1,2,4-oxadiazole,
4-(5-(1-Ethyl-6,6-dimethyl-4,5,6,7-tetrahydro-1H-indazol-3-yl)-1,2,4-oxadiazol-3-yl)-1-methylpyridin-2(1H)-one,
1-(4-(5-(1-Ethyl-6,6-dimethyl-4,5,6,7-tetrahydro-1H-indazol-3-yl)-1,2,4-oxadiazol-3-yl)phenyl)piperidine-4-carboxylic acid,
(4-(5-(1-Ethyl-6,6-dimethyl-4,5,6,7-tetrahydro-1H-indazol-3-yl)-1,2,4-oxadiazol-3-yl)phenyl)methanol,
4-(5-(6,6-Dimethyl-1-phenyl-4,5,6,7-tetrahydro-1H-indazol-3-yl)-1,2,4-oxadiazol-3-yl)benzamide,
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-ol,
5-(5-(1-Ethyl-6,6-dimethyl-4,5,6,7-tetrahydro-1H-indazol-3-yl)-1,2,4-oxadiazol-3-yl)-3-methylpyridin-2-ol,
4-(5-(1-Ethyl-6,6-dimethyl-4,5,6,7-tetrahydro-1H-indazol-3-yl)-1,2,4-oxadiazol-3-yl)-3-methylbenzamide,
4-(4-(5-(1-Ethyl-6,6-dimethyl-4,5,6,7-tetrahydro-1H-indazol-3-yl)-1,2,4-oxadiazol-3-yl)benzyl)morpholine,
3-Ethyl-5-(5-(1-ethyl-6,6-dimethyl-4,5,6,7-tetrahydro-1H-indazol-3-yl)-1,2,4-oxadiazol-3-yl)-1,6-dimethylpyridin-2(1H)-one,
5-(5-(1-Ethyl-6,6-dimethyl-4,5,6,7-tetrahydro-1H-indazol-3-yl)-1,2,4-oxadiazol-3-yl)-1,3-dimethylpyridin-2(1H)-one,
N-Cyclopropyl-4-(5-(1-ethyl-6,6-dimethyl-4,5,6,7-tetrahydro-1H-indazol-3-yl)-1,2,4-oxadiazol-3-yl)benzamide,
1-(4-(5-(1-Ethyl-6,6-dimethyl-4,5,6,7-tetrahydro-1H-indazol-3-yl)-1,2,4-oxadiazol-3-yl)phenyl)-N-methylmethanamine,
5-(1-Ethyl-6,6-dimethyl-4,5,6,7-tetrahydro-1H-indazol-3-yl)-3-(3-methylpyridin-4-yl)-1,2,4-oxadiazole,
4-(5-(6,6-Dimethyl-1-(2,2,2-trifluoroethyl)-4,5,6,7-tetrahydro-1H-indazol-3-yl)-1,2,4-oxad iazol-3-yl)benzam ide,
(4-(5-(1-Ethyl-6,6-dimethyl-4,5,6,7-tetrahydro-1H-indazol-3-yl)-1,2,4-oxadiazol-3-yl)phenyl)methanamine,
5-(1-Ethyl-6,6-dimethyl-4,5,6,7-tetrahydro-1H-indazol-3-yl)-3-(3-methylpyridin-2-yl)-1,2,4-oxadiazole,
2-(4-(5-(1-Ethyl-6,6-dimethyl-4,5,6,7-tetrahydro-1H-indazol-3-yl)-1,2,4-oxadiazol-3-yl)-2,6-dimethylphenyl)ethanamine,
5-(5-(1-Ethyl-6,6-dimethyl-4,5,6,7-tetrahydro-1H-indazol-3-yl)-1,2,4-oxadiazol-3-yl)-4-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-methylphenyl)propanoic acid,
5-(5-(1-Ethyl-6,6-dimethyl-4,5,6,7-tetrahydro-1H-indazol-3-yl)-1,2,4-oxadiazol-3-yl)-6-methylpyridin-2-ol,
5-(1-Ethyl-6,6-dimethyl-4,5,6,7-tetrahydro-1H-indazol-3-yl)-3-(4-methylpyridin-3-yl)-1,2,4-oxadiazole,
5-(1-Ethyl-6,6-dimethyl-4,5,6,7-tetrahydro-1H-indazol-3-yl)-3-(4-(trifluoromethyl)-pyridin-3-yl)-1,2,4-oxadiazole,
5-(1-Ethyl-6,6-dimethyl-4,5,6,7-tetrahydro-1H-indazol-3-yl)-3-(imidazo[1,2-a]pyridin-6-yl)-1,2,4-oxadiazole,
3-(4-(5-(1-Ethyl-6,6-dimethyl-4,5,6,7-tetrahydro-1H-indazol-3-yl)-1,2,4-oxadiazol-3-yl)-3-methylphenyl)propanamide,
2-(4-(5-(1-Ethyl-6,6-dimethyl-4,5,6,7-tetrahydro-1H-indazol-3-yl)-1,2,4-oxadiazol-3-yl)-3-methylphenyl)ethanamine,
3-(4-(5-(1-Ethyl-6,6-dimethyl-4,5,6,7-tetrahydro-1H-indazol-3-yl)-1,2,4-oxadiazol-3-yl)-2,6-dimethylphenyl)propan-1-amine,
4-(5-(1-Ethyl-6,6-dimethyl-4,5,6,7-tetrahydro-1H-indazol-3-yl)-1,2,4-oxadiazol-3-yl)-3-(trifluoromethyl)benzoic acid,
4-(5-(1-Ethyl-6,6-dimethyl-4,5,6,7-tetrahydro-1H-indazol-3-yl)-1,2,4-oxadiazol-3-yl)-3-(trifluoromethyl)benzamide,
5-(1-Ethyl-6,6-dimethyl-4,5,6,7-tetrahydro-1H-indazol-3-yl)-3-(2-methylpyridin-3-yl)-1,2,4-oxadiazole,
5-(5-(1-Ethyl-6,6-dimethyl-4,5,6,7-tetrahydro-1H-indazol-3-yl)-1,2,4-oxadiazol-3-yl)-6-(trifluoromethyl)pyridin-2-amine,
3-Cyclopropyl-4-[5-(1-ethyl-6,6-dimethyl-4,5,6,7-tetrahydro-1H-indazol-3-yl)-1,2,4-oxadiazol-3-yl]benzamide,
5-[5-(1-Ethyl-6,6-dimethyl-4,5,6,7-tetrahydro-1H-indazol-3-yl)-1,2,4-oxadiazol-3-yl]-6-(trifluoromethyl)pyridin-2-ol,
5-(5-(6,6-Dimethyl-1-(2,2,2-trifluoroethyl)-4,5,6,7-tetrahydro-1H-indazol-3-yl)-1,2,4-oxadiazol-3-yl)-3-ethyl-6-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanoic acid,
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propanamide,
3-Ethyl-5-[5-(1-ethyl-6,6-dimethyl-1,4,6,7-tetrahydropyrano[4,3-c]pyrazol-3-yl)-1,2,4-oxadiazol-3-yl]-6-methylpyridin-2-ol,
3-(4-(5-(1-Ethyl-6,6-dimethyl-4,5,6,7-tetrahydro-1H-indazol-3-yl)-1,2,4-oxadiazol-3-yl)-3-methylphenyl)propan-1-amine,
3-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)propan-1-amine,
6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-amine,
2-(4-(5-(1-Ethyl-6,6-dimethyl-4,5,6,7-tetrahydro-1H-indazol-3-yl)-1,2,4-oxadiazol-3-yl)-3-(trifluoromethyl)phenyl)ethanamine,
3-(2,6-Dimethyl-4-(5-(1,6,6-trimethyl-4,5,6,7-tetrahydro-1H-indazol-3-yl)-1,2,4-oxadiazol-3-yl)phenyl)propanoic acid,
3-(2,6-Dimethyl-4-(5-(1,6,6-trimethyl-4,5,6,7-tetrahydro-1H-indazol-3-yl)-1,2,4-oxadiazol-3-yl)phenyl)propanamide,
3-(4-(5-(6,6-Dimethyl-4,5,6,7-tetrahydrobenzo[d]isoxazol-3-yl)-1,2,4-oxadiazol-3-yl)-2,6-dimethylphenyl)propanoic acid,
3-(4-(5-(6,6-Dimethyl-4,5,6,7-tetrahydrobenzo[d]isoxazol-3-yl)-1,2,4-oxadiazol-3-yl)-2,6-dimethylphenyl)propanamide,
2-(6-(5-(1-Ethyl-6,6-dimethyl-4,5,6,7-tetrahydro-1H-indazol-3-yl)-1,2,4-oxadiazol-3-yl)-1,2,3,4-tetrahydronaphthalen-2-ylamino)ethanoic acid,
3-(2,6-Dimethyl-4-(5-(1,6,6-trimethyl-4,5,6,7-tetrahydro-1H-indazol-3-yl)-1,2,4-oxadiazol-3-yl)phenyl)propan-1-amine,
3-(4-(5-(6,6-Dimethyl-4,5,6,7-tetrahydrobenzo[d]isoxazol-3-yl)-1,2,4-oxadiazol-3-yl)-2,6-dimethylphenyl)propan-1-amine,
2-(2,6-Dimethyl-4-(5-(1,6,6-trimethyl-4,5,6,7-tetrahydro-1H-indazol-3-yl)-1,2,4-oxadiazol-3-yl)phenyl)ethanamine,
2-(4-(5-(6,6-Dimethyl-4,5,6,7-tetrahydrobenzo[d]isoxazol-3-yl)-1,2,4-oxadiazol-3-yl)-2,6-dimethylphenyl)ethanamine,
5-(5-(6,6-Dimethyl-1-(pyridin-3-ylmethyl)-4,5,6,7-tetrahydro-1H-indazol-3-yl)-1,2,4-oxadiazol-3-yl)-3-ethyl-6-methylpyridin-2(1H)-one,
2-(Dimethyl-4,5,6,7-tetrahydrobenzo[d]isoxazol-3-yl)-1,2,4-oxadiazol-3-yl)-2,6-dimethylphenethylamino)ethanoic acid,
2-(3-(4-(5-(6,6-Dimethyl-4,5,6,7-tetrahydrobenzo[d]isoxazol-3-yl)-1,2,4-oxadiazol-3-yl)-2,6-dimethylphenyl)propylamino)ethanoic acid,
3-(Dimethyl-4,5,6,7-tetrahydrobenzo[d]isoxazol-3-yl)-1,2,4-oxadiazol-3-yl)-2,6-dimethylphenethylamino)propanoic acid,
3-Ethyl-5-(5-(1-ethyl-4,5,6,7-tetrahydro-1H-indazol-3-yl)-1,2,4-oxadiazol-3-yl)-6-methylpyridin-2(1H)-one,
3-Ethyl-6-methyl-5-(5-(1,6,6-trimethyl-4,5,6,7-tetrahydro-1H-indazol-3-yl)-1,2,4-oxadiazol-3-yl)pyridin-2-ol,
5-(5-(6,6-Dimethyl-4,5,6,7-tetrahydrobenzo[d]isoxazol-3-yl)-1,2,4-oxadiazol-3-yl)-3-ethyl-6-methylpyridin-2(1 H)-one,
5-(5-(1-(2-Aminoethyl)-6,6-dimethyl-4,5,6,7-tetrahydro-1H-indazol-3-yl)-1,2,4-oxadiazol-3-yl)-3-ethyl-6-methylpyridin-2-ol,
3-Ethyl-5-(5-(1-ethyl-6,6-dimethyl-4,5,6,7-tetrahydro-1H-indazol-3-yl)-1,2,4-oxadiazol-3-yl)-6-methylpyridin-2-amine,
2-(4-(5-(6,6-Dimethyl-4,5,6,7-tetrahydrobenzo[d]isoxazol-3-yl)-1,2,4-oxadiazol-3-yl)-2,6-dimethylphenyl)-N,N-dimethylethanamine,
3-(4-(4-(5-(6,6-Dimethyl-4,5,6,7-tetrahydrobenzo[d]isoxazol-3-yl)-1,2,4-oxadiazol-3-yl)phenyl)piperazin-1-yl)propanoic acid,
3-Ethyl-5-(5-(1-ethyl-6,6-difluoro-4,5,6,7-tetrahydro-1H-indazol-3-yl)-1,2,4-oxadiazol-3-yl)-6-methylpyridin-2(1H)-one,
3-(3-Ethyl-5-(5-(1-ethyl-6,6-dimethyl-4,5,6,7-tetrahydro-1H-indazol-3-yl)-1,2,4-oxadiazol-3-yl)-6-methylpyridin-2-yl)propanoic acid,
5-(5-(1-(Cyclopropylmethyl)-6,6-dimethyl-4,5,6,7-tetrahydro-1H-indazol-3-yl)-1,2,4-oxadiazol-3-yl)-3-ethyl-6-methylpyridin-2(1H)-one
3-(2,6-Dimethyl-4-(5-(1,6,6-trimethyl-4,5,6,7-tetrahydro-1H-indazol-3-yl)-1,2,4-oxadiazol-3-yl)phenyl)propane-1,2-diol,
N-(2,6-Dimethyl-4-(5-(1,6,6-trimethyl-4,5,6,7-tetrahydro-1H-indazol-3-yl)-1,2,4-oxadiazol-3-yl)phenethyl)-2,2,2-trifluoroethanamine,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanol,
2-(2,6-Dimethyl-4-(5-(1,6,6-trimethyl-4,5,6,7-tetrahydro-1H-indazol-3-yl)-1,2,4-oxadiazol-3-yl)phenethylamino)ethanoic acid,
1-(2,6-Dimethyl-4-(5-(1,6,6-trimethyl-4,5,6,7-tetrahydro-1H-indazol-3-yl)-1,2,4-oxadiazol-3-yl)phenethyl)azetidine-3-carboxylic acid,
3-(2-Methyl-4-(5-(1,6,6-trimethyl-4,5,6,7-tetrahydro-1H-indazol-3-yl)-1,2,4-oxadiazol-3-yl)phenyl)propanoic acid,
4-(2,6-Dimethyl-4-(5-(1,6,6-trimethyl-4,5,6,7-tetrahydro-1H-indazol-3-yl)-1,2,4-oxadiazol-3-yl)phenethyl)morpholine,
3-(4-(5-(6,6-Dimethyl-4,5,6,7-tetrahydro-1H-indazol-3-yl)-1,2,4-oxadiazol-3-yl)-2,6-dimethylphenyl)propane-1,2-diol,
3-(4-(5-(1-Ethyl-6,6-dimethyl-4,5,6,7-tetrahydro-1H-indazol-3-yl)-1,2,4-oxadiazol-3-yl)phenylamido)propanoic acid,
3-(4-(5-(6,6-Dimethyl-1-(pyridin-3-ylmethyl)-4,5,6,7-tetrahydro-1H-indazol-3-yl)-1,2,4-oxadiazol-3-yl)-2,6-dimethylphenyl)propane-1,2-diol,
3-(2-Chloro-4-(5-(1-ethyl-6,6-dimethyl-4,5,6,7-tetrahydro-1H-indazol-3-yl)-1,2,4-oxadiazol-3-yl)phenylsulfonamido)propanoic acid,
3-(4-(5-(6,6-Dimethyl-4,5,6,7-tetrahydrobenzo[d]isoxazol-3-yl)-1,2,4-oxadiazol-3-yl)-2,6-dimethylphenyl)propane-1,2-diol,
3-(1-Ethyl-6,6-dimethyl-4,5,6,7-tetrahydro-1H-indazol-3-yl)-5-(pyridin-4-yl)-1,2,4-oxadiazole,
5-(1-Ethyl-6,6-dimethyl-4,5,6,7-tetrahydro-1H-indazol-3-yl)-3-o-tolyl-1,2,4-oxadiazole,
3-(5-(5-(1-Ethyl-6,6-dimethyl-4,5,6,7-tetrahydro-1H-indazol-3-yl)-1,2,4-oxadiazol-3-yl)-1H-pyrrolo[2,3-b]pyridin-1-yl)propanoic acid,
3-(5-(5-(6,6-Dimethyl-4,5,6,7-tetrahydrobenzo[d]isoxazol-3-yl)-1,2,4-oxadiazol-3-yl)-1 H-pyrrolo[2,3-b]pyridin-1-yl)propanoic acid,
1-amino-3-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylpropan-2-amine,
3-{4-[3-(1-ethyl-6,6-dimethyl-4,5,6,7-tetrahydro-1H-indazol-3-yl)-1,2,4-oxadiazol-5-yl]-3-methylphenyl}propanoic acid,
[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]amine,
3-(4-{5-[1-(cyclopropylmethyl)-6,6-dimethyl-4,5,6,7-tetrahydro-1H-indazol-3-yl]-1,2,4-oxadiazol-3-yl}-2,6-dimethylphenyl)propanoic acid,
(2-{4-[3-(1-ethyl-6,6-dimethyl-4,5,6,7-tetrahydro-1H-indazol-3-yl)-1,2,4-oxadiazol-5-yl]-3-methylphenyl}ethyl)amine,
N-[2-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)ethyl]glycine,
3-{4-[5-(1-ethyl-6,6-difluoro-4,5,6,7-tetrahydro-1H-indazol-3-yl)-1,2,4-oxadiazol-3-yl]-2,6-dimethylphenyl}propanoic acid,
3-(4-{5-[6,6-dimethyl-1-(pyridin-3-ylmethyl)-4,5,6,7-tetrahydro-1H-indazol-3-yl]-1,2,4-oxadiazol-3-yl}-2,6-dimethylphenyl)propanoic acid,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-hydroxyacetamide,
3-{4-[5-(1-ethyl-6,6-dimethyl-4,5,6,7-tetrahydro-1H-indazol-3-yl)-1,2,4-oxadiazol-3-yl]-2,6-dimethylphenyl}-N-(methylsulfonyl)propanamide,
3-{4-[5-(1-ethyl-6,6-dimethyl-4,5,6,7-tetrahydro-1H-indazol-3-yl)-1,2,4-oxadiazol-3-yl]-2-methylphenyl}propanoic acid,
3-{4-[5-(1-ethyl-6,6-dimethyl-4,5,6,7-tetrahydro-1H-indazol-3-yl)-1,2,4-oxadiazol-3-yl]-2,6-dimethylphenyl}propane-1,2-diol,
N-(2-{4-[5-(6,6-dimethyl-4,5,6,7-tetrahydro-1H-indazol-3-yl)-1,2,4-oxadiazol-3-yl]-2,6-dimethylphenyl}ethyl)-2,2,2-trifluoroethanamine,
N-({4-[5-(1-ethyl-6,6-dimethyl-4,5,6,7-tetrahydro-1H-indazol-3-yl)-1,2,4-oxadiazol-3-yl]-3-methylphenyl}sulfonyl)-beta-alamine,
N-(3-{4-[5-(1-ethyl-6,6-dimethyl-4,5,6,7-tetrahydro-1H-indazol-3-yl)-1,2,4-oxadiazol-3-yl]-2,6-dimethylphenyl}propyl)methanesulfonamide,
N-(3-{2,6-dimethyl-4-[5-(1,6,6-trimethyl-4,5,6,7-tetrahydro-1H-indazol-3-yl)-1,2,4-oxadiazol-3-yl]phenyl}propanoyl)glycine,
(2-{4-[5-(1-ethyl-6,6-dimethyl-4,5,6,7-tetrahydro-1H-indazol-3-yl)-1,2,4-oxadiazol-3-yl]-2-methylphenyl}ethyl)amine,
N-({4-[5-(1-ethyl-6,6-dimethyl-4,5,6,7-tetrahydro-1H-indazol-3-yl)-1,2,4-oxadiazol-3-yl]-3-methylphenyl}sulfonyl)glycine,
1-ethyl-6,6-dimethyl-3-[3-(2-methyl-4-piperidin-4-ylphenyl)-1,2,4-oxadiazol-5-yl]-4,5,6,7-tetrahydro-1 H-indazole,
(4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)acetic acid,
3-(4-{4-[5-(1-ethyl-6,6-dimethyl-4,5,6,7-tetrahydro-1H-indazol-3-yl)-1,2,4-oxadiazol-3-yl]-3-methylphenyl}piperidin-1-yl)propanoic acid,
(2-{4-[3-(1-ethyl-6,6-dimethyl-4,5,6,7-tetrahydro-1H-indazol-3-yl)-1,2,4-oxadiazol-5-yl]-2,6-dimethylphenoxy}ethyl)amine,
3-{4-[5-(1-ethyl-6,6-dimethyl-4,5,6,7-tetrahydro-1H-indazol-3-yl)-1,2,4-oxadiazol-3-yl]-2,5-dimethylphenyl}propanoic acid,
{4-[3-(1-ethyl-6,6-dimethyl-4,5,6,7-tetrahydro-1H-indazol-3-yl)-1,2,4-oxadiazol-5-yl]-2,6-dimethylphenoxy}acetic acid,
3-{4-[3-(1-ethyl-6,6-dimethyl-4,5,6,7-tetrahydro-1H-indazol-3-yl)-1,2,4-oxadiazol-5-yl]-2,6-dimethoxyphenyl}propanoic acid,
3-{2-chloro-4-[3-(1-ethyl-6,6-dimethyl-4,5,6,7-tetrahydro-1H-indazol-3-yl)-1,2,4-oxadiazol-5-yl]-6-methoxyphenyl}propanoic acid,
(2-{2,6-dimethyl-4-[5-(1,6,6-trimethyl-4,5,6,7-tetrahydro-1H-indazol-3-yl)-1,2,4-oxadiazol-3-yl]phenyl}ethyl)[2-(methylsulfonyl)ethyl]amine,
3-{2-ethyl-4-[5-(1-ethyl-6,6-dimethyl-4,5,6,7-tetrahydro-1H-indazol-3-yl)-1,2,4-oxadiazol-3-yl]-6-methylphenyl}propanoic acid,
(2-{3-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
{4-[5-(1-ethyl-6,6-dimethyl-4,5,6,7-tetrahydro-1H-indazol-3-yl)-1,2,4-oxadiazol-3-yl]-2,6-dimethylphenyl}acetic acid,
[3-({3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}oxy)propyl]amine,
1,6,6-trimethyl-3-[3-(2-methyl-4-piperidin-4-ylphenyl)-1,2,4-oxadiazol-5-yl]-4,5,6,7-tetrahydro-1 H-indazole,
2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
2-{4-[5-(1-ethyl-6,6-dimethyl-4,5,6,7-tetrahydro-1H-indazol-3-yl)-1,2,4-oxadiazol-3-yl]-2,6-dimethylphenyl}acetamide,
(2-{3-ethyl-5-[5-(1-ethyl-6,6-dimethyl-4,5,6,7-tetrahydro-1H-indazol-3-yl)-1,2,4-oxadiazol-3-yl]-6-methylpyridin-2-yl}ethyl)amine,
(2-{2,6-dimethyl-4-[5-(1,6,6-trimethyl-4,5,6,7-tetrahydro-1H-indazol-3-yl)-1,3,4-thiadiazol-2-yl]phenoxy}ethyl)amine,
{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}amine,
2-{2,6-dimethyl-4-[5-(1,6,6-trimethyl-4,5,6,7-tetrahydro-1H-indazol-3-yl)-1,2,4-oxadiazol-3-yl]phenyl}ethanol,
1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
[1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidin-4-yl]acetic acid,
1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid,
(3S)-1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)pyrrolidine-3-carboxylic acid, N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-L-alanine,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-2-methylalanine,
N-(2-{2,6-dimethyl-4-[5-(1,6,6-trimethyl-4,5,6,7-tetrahydro-1H-indazol-3-yl)-1,2,4-oxadiazol-3-yl]phenyl}ethyl)-D-alanine,
2-((2,6-dimethyl-4-(5-(1,6,6-trimethyl-4,5,6,7-tetrahydro-1H-indazol-3-yl)-1,2,4-oxadiazol-3-yl)phenethyl)(methyl)amino)acetic acid,
(2-{2,6-dimethyl-4-[5-(1,6,6-trimethyl-4,5,6,7-tetrahydro-1H-indazol-3-yl)-1,3,4-oxadiazol-2-yl]phenoxy}ethyl)amine,
{2-[4-[5-(1-ethyl-6,6-dimethyl-4,5,6,7-tetrahydro-1H-indazol-3-yl)-1,2,4-oxadiazol-3-yl]-2-(trifluoromethyl)phenoxy]ethyl}amine,
(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)amine,
(2-{2-(trifluoromethyl)-4-[5-(1,6,6-trimethyl-4,5,6,7-tetrahydro-1H-indazol-3-yl)-1,2,4-oxadiazol-3-yl]phenoxy}ethyl)amine,
(2,2-difluoro-2-{2-methyl-4-[5-(1,6,6-trimethyl-4,5,6,7-tetrahydro-1H-indazol-3-yl)-1,2,4-oxadiazol-3-yl]phenyl}ethyl)amine,
1-{2,6-dimethyl-4-[5-(1,6,6-trimethyl-4,5,6,7-tetrahydro-1H-indazol-3-yl)-1,2,4-oxadiazol-3-yl]phenyl}propan-2-ol,
3-{2,6-dimethyl-4-[5-(1,6,6-trimethyl-4,5,6,7-tetrahydro-1H-indazol-3-yl)-1,2,4-oxadiazol-3-yl]phenyl}propan-1-ol,,
[4-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperazin-1-yl]acetic acid,
1-(2-{4-[5-(1-ethyl-6,6-dimethyl-4,5,6,7-tetrahydro-1H-indazol-3-yl)-1,2,4-oxadiazol-3-yl]-2,6-dimethylphenyl}ethyl)piperidine-4-carboxylic acid,
1-(2-{4-[5-(1-ethyl-6,6-dimethyl-4,5,6,7-tetrahydro-1H-indazol-3-yl)-1,2,4-oxadiazol-3-yl]-2-methylphenyl}ethyl)piperidine-4-carboxylic acid,
1-(2-{4-[3-(1-ethyl-6,6-dimethyl-4,5,6,7-tetrahydro-1H-indazol-3-yl)-1,2,4-oxadiazol-5-yl]-2-methylphenyl}ethyl)piperidine-4-carboxylic acid,
1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidine-4-carboxylic acid, N-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}glycine,
4-[5-(1,6,6-trimethyl-4,5,6,7-tetrahydro-1H-indazol-3-yl)-1,2,4-oxadiazol-3-yl]benzoic acid,
1-(2-{3-methyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid,
(1-{2-[4-[5-(1-ethyl-6,6-dimethyl-4,5,6,7-tetrahydro-1H-indazol-3-yl)-1,2,4-oxadiazol-3-yl]-2-(trifluoromethyl)phenyl]ethyl}piperidin-4-yl)acetic acid, 2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethanol,
N-{2-[(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)amino]ethyl}methane-sulfonamide, N-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)-2,2,2-trifluoroethanamine,
1-(2-{4-[5-(6,6-dimethyl-4,5,6,7-tetrahydro-1,2-benzisoxazol-3-yl)-1,2,4-oxadiazol-3-yl]-2,6-dimethylphenyl}ethyl)piperidine-4-carboxylic acid,
3-{4-[5-(6,6-dimethyl-4,5,6,7-tetrahydro-1,2-benzisoxazol-3-yl)-1,2,4-oxadiazol-3-yl]-2,6-dimethylphenyl}propane-1,2-diol,
1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid, 2-(2-(trifluoromethyl)-4-(5-(1,6,6-trimethyl-4,5,6,7-tetrahydro-1H-indazol-3-yl)-1,2,4-oxadiazol-3-yl)phenethylamino)acetic acid,
2-(methyl(2-(trifluoromethyl)-4-(5-(1,6,6-trimethyl-4,5,6,7-tetrahydro-1H-indazol-3-yl)-1,2,4-oxadiazol-3-yl)phenethyl)amino)acetic acid,
1-(2-(trifluoromethyl)-4-(5-(1,6,6-trimethyl-4,5,6,7-tetrahydro-1H-indazol-3-yl)-1,2,4-oxadiazol-3-yl)phenethyl)azetidine-3-carboxylic acid,
2-(1-(2-(trifluoromethyl)-4-(5-(1,6,6-trimethyl-4,5,6,7-tetrahydro-1H-indazol-3-yl)-1,2,4-oxadiazol-3-yl)phenethyl)piperidin-4-yl)acetic acid,
1-(2,6-dimethyl-4-(5-(1,6,6-trimethyl-4,5,6,7-tetrahydro-1H-indazol-3-yl)-4H-1,2,4-triazol-3-yl)phenethyl)piperidine-4-carboxylic acid,
1-(2,6-dimethyl-4-(5-(1,6,6-trimethyl-4,5,6,7-tetrahydro-1H-indazol-3-yl)-1,3,4-oxadiazol-2-yl)phenethyl)piperidine-4-carboxylic acid,
1-(2,6-dimethyl-4-(5-(1,6,6-trimethyl-4,5,6,7-tetrahydro-1H-indazol-3-yl)-1,3,4-thiadiazol-2-yl)phenethyl)piperidine-4-carboxylic acid,
2-(1-(2,6-dimethyl-4-(5-(1,6,6-trimethyl-4,5,6,7-tetrahydro-1H-indazol-3-yl)-1,3,4-thiadiazol-2-yl)phenethyl)piperidin-4-yl)acetic acid,
1-(2-(trifluoromethyl)-4-(5-(1,6,6-trimethyl-4,5,6,7-tetrahydro-1H-indazol-3-yl)-1,3,4-oxadiazol-2-yl)phenethyl)piperidine-4-carboxylic acid,
1-(2-(trifluoromethyl)-4-(5-(1,6,6-trimethyl-4,5,6,7-tetrahydro-1H-indazol-3-yl)-1,3,4-thiadiazol-2-yl)phenethyl)piperidine-4-carboxylic acid,
2-(1-(2-(trifluoromethyl)-4-(5-(1,6,6-trimethyl-4,5,6,7-tetrahydro-1H-indazol-3-yl)-1,3,4-thiadiazol-2-yl)phenethyl)piperidin-4-yl)acetic acid,
2-(5-(5-(1-ethyl-6,6-dimethyl-4,5,6,7-tetrahydro-1H-indazol-3-yl)-1,2,4-oxadiazol-3-yl)-1H-pyrrolo[2,3-b]pyridin-1-yl)ethanamine,
5-(1-ethyl-6,6-dimethyl-4,5,6,7-tetrahydro-1H-indazol-3-yl)-3-(1H-pyrrolo[2,3-b]pyridin-5-yl)-1,2,4-oxadiazole,
2-(5-(5-(1,6,6-trimethyl-4,5,6,7-tetrahydro-1H-indazol-3-yl)-1,2,4-oxadiazol-3-yl)-1H-pyrrolo[2,3-b]pyridin-1-yl)ethanamine,
3-(1H-indazol-5-yl)-5-(1,6,6-trimethyl-4,5,6,7-tetrahydro-1H-indazol-3-yl)-1,2,4-oxadiazole,
5-(1-ethyl-6,6-dimethyl-4,5,6,7-tetrahydro-1H-indazol-3-yl)-3-(1H-indol-4-yl)-1,2,4-oxadiazole,
3-(1H-indol-4-yl)-5-(1,6,6-trimethyl-4,5,6,7-tetrahydro-1H-indazol-3-yl)-1,2,4-oxadiazole,
5-(1-ethyl-6,6-dimethyl-4,5,6,7-tetrahydro-1H-indazol-3-yl)-3-(1H-indol-5-yl)-1,2,4-oxadiazole,
3-(1H-benzo[d]imidazol-5-yl)-5-(1-ethyl-6,6-dimethyl-4,5,6,7-tetrahydro-1H-indazol-3-yl)-1,2,4-oxadiazole,
2-(4-(5-(1-ethyl-6,6-dimethyl-4,5,6,7-tetrahydro-1H-indazol-3-yl)-1,2,4-oxadiazol-3-yl)-1H-indol-1-yl)acetic acid,
2-(5-(5-(1,6,6-trimethyl-4,5,6,7-tetrahydro-1H-indazol-3-yl)-1,2,4-oxadiazol-3-yl)-1H-indol-1-yl)acetic acid,
3-(4-(5-(1-ethyl-6,6-dimethyl-4,5,6,7-tetrahydro-1H-indazol-3-yl)-1,2,4-oxadiazol-3-yl)-1H-indol-1-yl)propanoic acid,
2-(5-(5-(1-ethyl-6,6-dimethyl-4,5,6,7-tetrahydro-1H-indazol-3-yl)-1,2,4-oxadiazol-3-yl)-1H-indol-1-yl)acetic acid,
3-(5-(5-(1-ethyl-6,6-dimethyl-4,5,6,7-tetrahydro-1H-indazol-3-yl)-1,2,4-oxadiazol-3-yl)-1H-indol-1-yl)propanoic acid,
2-(1-(2-(trifluoromethyl)-4-(5-(1,6,6-trimethyl-4,5,6,7-tetrahydro-1H-indazol-3-yl)-1,3,4-oxadiazol-2-yl)phenethyl)piperidin-4-yl)acetic acid,
2-(2-(trifluoromethyl)-4-(5-(1,6,6-trimethyl-4,5,6,7-tetrahydro-1H-indazol-3-yl)-1,3,4-thiadiazol-2-yl)phenethylamino)acetic acid,
1-(2-(trifluoromethyl)-4-(5-(1,6,6-trimethyl-4,5,6,7-tetrahydro-1H-indazol-3-yl)-1,3,4-thiadiazol-2-yl)phenethyl)azetidine-3-carboxylic acid,
1-(2-(trifluoromethyl)-4-(3-(1,6,6-trimethyl-4,5,6,7-tetrahydro-1H-indazol-3-yl)-1,2,4-oxadiazol-5-yl)phenethyl)piperidine-4-carboxylic acid
2-(1-(2-(trifluoromethyl)-4-(3-(1,6,6-trimethyl-4,5,6,7-tetrahydro-1H-indazol-3-yl)-1,2,4-oxadiazol-5-yl)phenethyl)piperidin-4-yl)acetic acid
3-(3-(trifluoromethyl)-4-(5-(1,6,6-trimethyl-4,5,6,7-tetrahydro-1H-indazol-3-yl)-1,2,4-oxadiazol-3-yl)phenyl)propanoic acid,
3-(4-(5-(1-ethyl-6,6-dimethyl-4,5,6,7-tetrahydro-1H-indazol-3-yl)-1,2,4-oxadiazol-3-yl)-2-(trifluoromethyl)phenyl)propanoic acid, and
3-(2-(trifluoromethyl)-4-(5-(1,6,6-trimethyl-4,5,6,7-tetrahydro-1H-indazol-3-yl)-1,2,4-oxadiazol-3-yl)phenyl)propanoic acid
or a pharmaceutically acceptable salt, or solvate, or N-oxide, or stereoisomer or
deuterated derivate thereof.

22. The S1P1 receptor agonist according to claim 21 which is 1-(2-{2,6-dimethyl-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

23. The S1 P1 receptor agonist according to claim 22, wherein the pharmaceutically acceptable salt is hydrochloride.

24. The S1 P1 receptor agonist according to claim 21 which is 1-(2-{2-(trifluoromethyl)-4-[5-(1,6,6-trimethyl-4,5,6,7-tetrahydro-1H-indazol-3-yl)-1,2,4-oxadiazol-3-yl]phenyl}ethyl)piperidine-4-carboxylic acid or a pharmaceutically acceptable salt, or solvate, or N-oxide, or stereoisomer or deuterated derivate thereof.

25. The S1 P1 receptor agonist according to claim 24, wherein the pharmaceutically acceptable salt is hydrochloride.

26. A combination comprising (a) IFNβ and (b) a S1P1 receptor agonist of formula (I) as defined in any one of claims 1 to 25.

27. A combination according to claim 26 for use in the treatment of multiple sclerosis.

28. A combination according to claim 26 or 27, which further comprises (c) another compound selected from:
a) Immunomodulators such as glatiramer acetate
b) Inhibitors of DNA synthesis and repair, such as Mitoxantrone
c) Anti-alpha 4 integrin antibodies, such as Natalizumab (Tysabri)
d) Alpha 4 integrin antagonists such as R-1295 , TBC-4746, CDP-323, ELND-002, Firategrast and TMC-2003
e) Dyhydrofolate reductase inhibitors, such as Methotrexate or CH-1504
f) Glucocorticoids such as prednisone or methylprednisolone
g) DHODH inhibitors such as teriflunomide and compounds disclosed in WO2008/077639 and WO2009/021696,
h) Fumaric acid esters, such as *BG-12*
i) Immunomodulators such as Laquinimod
j) Anti-CD20 monoclonal antibodies such as Rituximab, Ocrelizumab Ofatumumab or TRU-015
k) Anti-CD52 such as alemtuzumab
l) Anti-CD25 such as daclizumab
m) Anti-CD88, such as eculizumab or pexilizumab
n) Calcineurin inhibitors such as cyclosporine A or tacrolimus
o) IMPDH inhibitors, such as mycophenolate mophetyl
p) Cannabinoid receptor agonists such as Sativex
q) Chemokine CCR1 antagonists such as MLN-3897 or PS-031291
r) Chemokine CCR2 antagonists such as INCB-8696
s) Interferon alpha such as Sumiferon MP
t) NF-kappaB activation inhibitors such as FAE and MLN-0415
u) JAK inhibitors such as CP-690550 or INCB018424
v) Syk inhibitors, such as R-112
w) PKC inhibitors, such as NVP-AEB071
x) Phosphosdiesterase IV inhibitors such as GRC-4039
y) P38 Inhibitors such as ARRY-797
z) MEK inhibitors, such as ARRY-142886 or ARRY-438162

29. Use of a compound of formula (I) as defined in any one of claims 1-25, or a combination as defined in any one of claims 26-28 in the manufacture of a medicament for use in the treatment of multiple sclerosis.

30. A product comprising (a) IFNβ and (b) a compound of formula (I) as defined in any one of claims 1 to 25, as a combined preparation for simultaneous, separate or sequential use in the treatment of multiple sclerosis.

31. A product according to claim 30, which further comprises an active compound (c), as defined in claim 28.

32. A kit of parts comprising (b) a compound of formula (I) as defined in any one of claims 1 to 25 together with instructions for simultaneous, separate or sequential use in combination with (a) IFNβ for the treatment of multiple sclerosis.

33. A kit according to claim 32, which further comprises an active compound (c), as defined in claim 28.

34. A package comprising (b) a compound of formula (I) as defined in any one of claims 1 to 25 and (a) IFNβ for simultaneous, separate or sequential use in the treatment of a multiple scleroris.

35. A package according to claim 34, which further comprises an active compound (c), as defined in claim 28.

36. Use of (b) a compound of formula (I) as defined in any one of claims 1 to 25 for the preparation of a medicament, for use in combination with (a) IFNβ, for the treatment of multiple sclerosis.

37. Use of (a) IFNβ, for the preparation of a medicament for use in combination with (b) a compound of formula (I) as defined in any one of claims 1 to 25 for the treatment of multiple sclerosis.

38. Use according to claim 29, 36 or 37, wherein the IFNβ is for administration at a dosage regime which involves administration of 0.02 to 0.3 mg/week of IFNβ, and the compound of formula (I) is for administration at a dosage regime which involves administration of 0.5 to 2000 mg/day of compound of formula (I).

39. Use according to any one of claims 29 and 36 to 38, wherein the medicament is for use in treating a human or animal patient suffering from or susceptible to hepatic impairment or a condition that would be aggravated by hepatotoxicity.

40. Use according to claim 39, wherein said condition that would be aggravated by hepatotoxicity is liver fibrosis, hepatitis, cirrhosis or liver cancer.

41. A method of treating multiple sclerosis, which method comprises simultaneously, separately or sequentially administering to a human or animal patient a therapeutically effective amount of (a) IFNβ and (b) a compound of formula(I) as defined in any one of claims 1 to 25.

42. A method of treating a human or animal patient suffering from or susceptible to multiple sclerosis, wherein the human or animal patient is suffering from or susceptible to hepatic impairment or a condition that would be aggravated by hepatotoxicity as defined in claim 39 or 40, which method comprises administering to said human or animal patient a therapeutically effective amount of a compound of formula (I) as defined in any one of claims 1 to 25.
